# EUROPEAN PATENT SPECIFICATION

(11) **EP 2 296 669 B1**
(45) Date of publication and mention of the grant of the patent: **21.03.2012**
(21) Application number: 09767411.3
(22) Date of filing: 29.05.2009
(51) Int. Cl.: A61K 31/7088, C12N 15/113

(54) **TARGETED OLIGONUCLEOTIDE COMPOSITIONS FOR MODIFYING GENE EXPRESSION**
GEZIELTE OLIGONUCLEOTID-ZUSAMMENSETZUNGEN ZUR MODIFIZIERUNG DER GENEXPRESSION
COMPOSITIONS OLIGONUCLÉOTIDIQUES CIBLÉES POUR MODIFIER L'EXPRESSION GÉNIQUE

(30) Priority: 30.05.2008 US 130528 P
(43) Date of publication of application: 23.03.2011
(73) Proprietor: Yale University, New Haven, CT 06510 (US)
(72) Inventor: SLACK, Frank, J., Branford CT 06405 (US); WEIDHAAS, Joanne, B., Westport CT 06880 (US)
(74) Representative: Finnie, Isobel Lara
(86) International application number: PCT/US2009/045648
(87) International publication number: WO 2009/155100

(56) References cited:
- WO-A-2004/042027
- WO-A-2008/061537
- WO-A-2008/095096
- WO-A-2008/151004
- FLUITER K ET AL: "Killing cancer by targeting genes that cancer cells have lost: allele-specific inhibition, a novel approach to the treatment of genetic disorders" CMLS CELLULAR AND MOLECULAR LIFE SCIENCES, BIRKHAUSER VERLAG, HEIDELBERG, DE, vol. 60, no. 5, 1 May 2003 (2003-05-01), pages 834-843, XP002985258 ISSN: 1420-682X
- MILLER V M ET AL: "Allele-specific silencing of dominant disease genes" PROCEEDINGS OF THE NATIONAL ACADEMY OF SCIENCES OF USA, NATIONAL ACADEMY OF SCIENCE, WASHINGTON, DC, US, vol. 100, no. 12, 10 June 2003 (2003-06-10), pages 7195-7200, XP002484838 ISSN: 0027-8424
- GRUNWELLER A ET AL: "Locked nucleic acid oligonucleotides: The next generation of antisense agents?" BIODRUGS 2007 NZ, vol. 21, no. 4, 2007, pages 235-243, XP009125276 ISSN: 1173-8804

## Description

### FIELD OF THE INVENTION

This invention relates generally to the fields of cancer and molecular biology. The invention provides compositions for modifying the expression of genes that cause a variety of disorders, such as cancer.

### BACKGROUND OF THE INVENTION

Cancer is often caused by novel mutations that occur within regulatory sequences of genes controlling essential cellular functions such as DNA repair, cell cycle, proliferation, and cell adhesion. Current therapies are unable to address the underlying genetic mechanisms that lead to the development of the cancer. As such, there exists a long-felt need for a new kind of cancer therapy that is individualized and that addresses the underlying genetic and molecular mechanisms operating in cancer cells.

### SUMMARY OF THE INVENTION

The compositions and methods of the disclosure provide a new form of genetic and molecular therapy capable of treating cancer. Pharmaceutical compositions of the disclosure are individualized and optimized to target misregulated genes and/or specific mutations present in the genetic and epigenetic backgrounds of the intended subject. In one aspect of the disclosure, therapeutic compositions restore functions that are lost when novel or inherited mutations in miRNA binding sites modify wild type miRNA binding efficacies such that target genes are no longer sufficiently silenced in order to prevent the development of cancer.

In accordance with the invention, there is provided an isolated modified oligo, wherein said modified oligo comprises the nucleotide sequence of SEQ ID NO: 22, 24, 25, 26, 27, 29, 30, 31, 32, 34, 35 or 36.

The invention also provides a composition containing the above isolated modified oligo. In certain embodiments, compositions including isolated modified oligos also include a pharmaceutically acceptable carrier. In other embodiments of the invention, this composition further comprises a cytotoxic compound. Exemplary cytotoxic compounds include, but are not limited to, all radioactive isotopes and chemotherapeutic compounds.

The invention further provides a composition comprising an isolated modified oligo, wherein said modified oligo comprises the nucleotide sequence of SEQ ID NO: 22, 24, 25, 26, 27, 29, 30, 31, 32, 34, 35 or 36, for use in the treatment of cancer.

The disclosure provides a method of treating or alleviating a symptom of a cell proliferative disorder comprising administering to a subject in need thereof a composition of the invention. Preferably, the cell proliferative disorder is cancer. Accordingly, the disclosure provides a method of treating or alleviating a symptom of cancer comprising administering to a subject in need thereof a composition of the invention. Exemplary cancers include, but are not limited to, all varieties of lung cancer, ovarian cancer, breast cancer, uterine cancer, head and neck cancer, pancreatic cancer, prostate cancer, renal cancer or colon cancer. Preferably, the subject carries the LSC6 SNP.

Compositions are administered locally using the methods provided herein. Alternatively, or in addition, compositions are administered systemically using the methods provided herein. Compositions are administered intravenously, intradermally, subcutaneously, orally, transdermally, topically, transmucosally, transopthalmically, intratracheally, intranasally, epidermally, intraperitoneally, intraorbitally, intraarterially, intracapsularly, intraspinally, intrasternally, intracranially, intrathecally, intraventricularly, parenterally, non-parenterally, rectally, or surgically. Furthermore, multiple compositions may be administered to the same subject, either sequentially or simultaneously by any of the listed or art-recognized methods.

The disclosure provides methods of inducing cell death in a cancer cell comprising contacting a modified oligo composition to the cancer cell. The methods herein encompass cancer cells that are *in vivo*, *in vitro*, *ex vivo*, and *in situ*. Cancer cells of the methods include cells from all varieties of cancer including, but not limited to, lung cancer, ovarian cancer, breast cancer, uterine cancer, head and neck cancer, pancreatic cancer, prostate cancer, renal cancer, or colon cancer. In one aspect of the disclosure, the cancer cell contacted using the methods herein contains a mutation. Preferably, the mutation is the LCS6 SNP. Alternatively, the cancer cell contains the LCS6 SNP.

Compositions of the invention are contacted to a cancer cell using the methods disclosed herein by local or systemic administration. Compositions contact a cancer cell following, for example, topical, intravenous, intraocular, subcutaneous, intraparitoneal, intramuscular, intraspinal, or surgical administration.

### BRIEF DESCRIPTION OF THE DRAWINGS

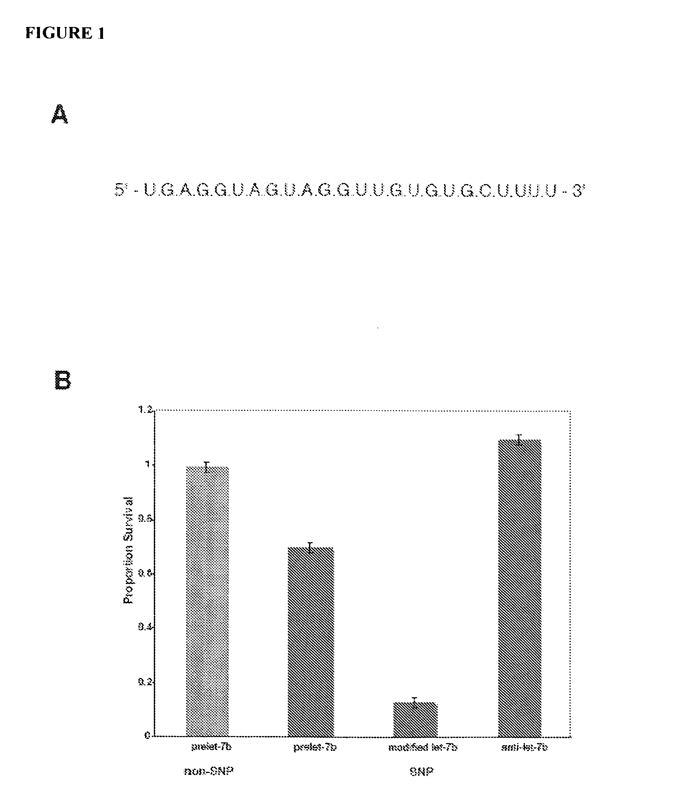

**Figure 1A** is the sequence of the modified oligo (SEQ ID NO: 22) used in Figure 1B.
**Figure 1B** is a graph showing the proportional survival of cancer cells, wild type (left two columns) or mutant (containing the LCS6 SNP mutation, right two columns) following exposure to wild type (prelet-7b), modified *let-7b* oligo, or antisense *let-7* (anti-*let-7*) miRNAs.

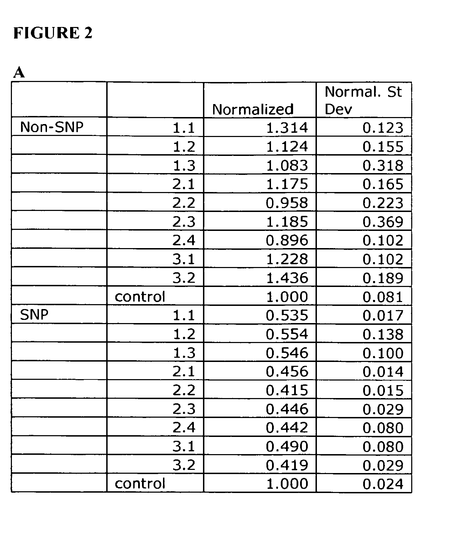

**Figure 2A** is a table showing the values of normalized luciferase expression and the standard deviations of the mean for each of three modified oligos when luciferase reporter proteins are fused to either wild type and LCS6 SNP containing KRAS 3'UTR regions. Values are normalized to a control miRNA construct such that the expression of the control is equal to 1.

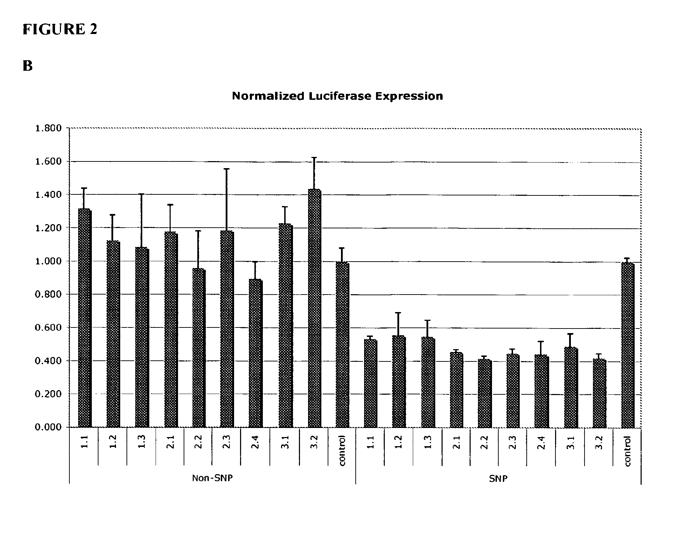

**Figure 2B** is a graphic representation of the values presented in Figure 2A.

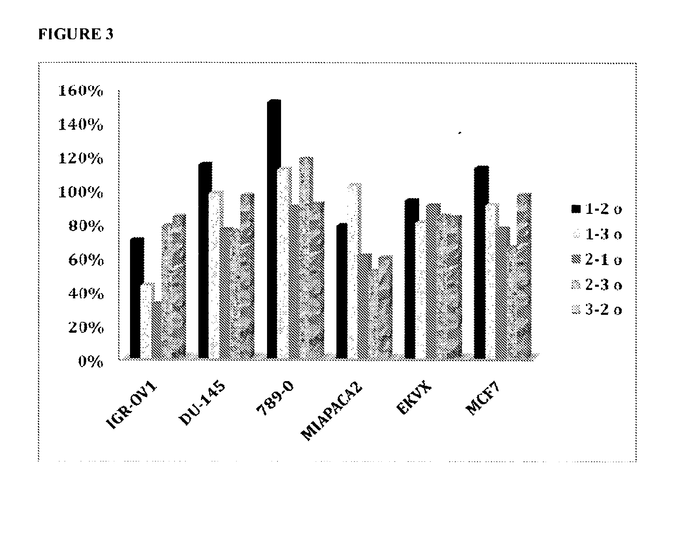

**Figure 3** is a graphic representation of a clonogenic assay demonstrating the ability of different combinations of oligonucleotides (1-2, 1-3, 2-1, 2-3, and 3-2) to silence KRAS expression, and therefore, affect cell survival of oncogenic cell lines containing the LCS6 SNP (also referred to as the onco-SNP).

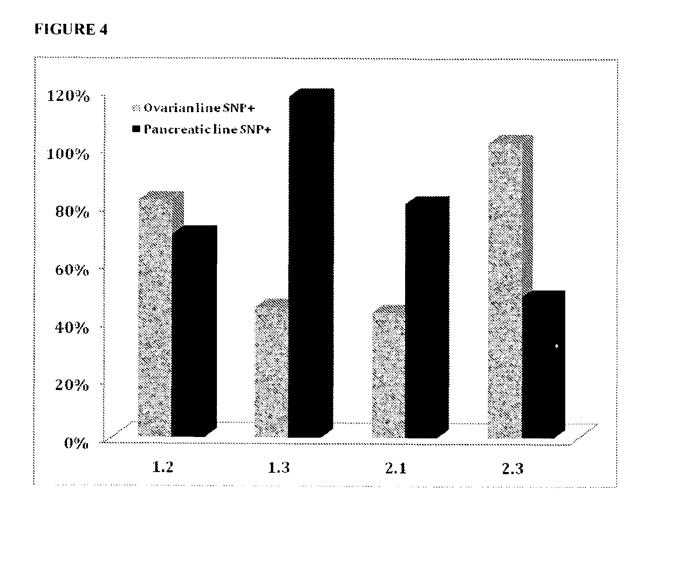

**Figure 4** is a graphic representation of a clonogenic assay demonstrating the ability of different combinations of oligonucleotides (1.2, 1.3, 2.1, and 2.3) to silence KRAS expression, and therefore, affect cell survival of ovarian and pancreatic cancer cell lines containing the LCS6 SNP. Cell survival was normalized to ovarian and pancreatic cancer cell lines that do not contain the LCS6 SNP and expressed as a percentage of the survival observed in the non-SNP cell lines.

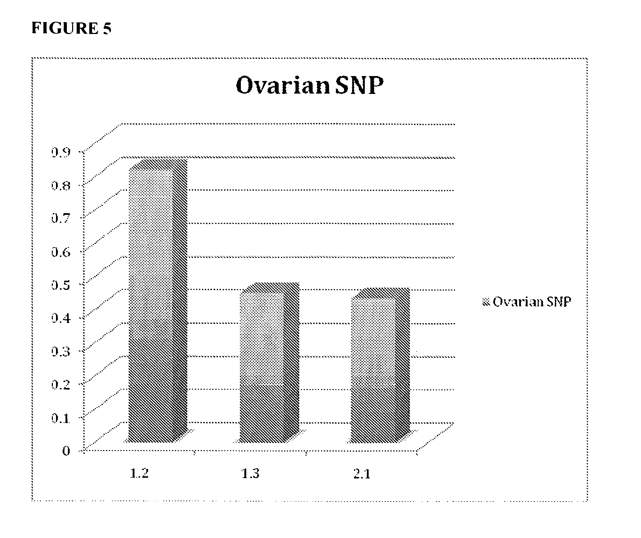

**Figure 5** is a graphic representation of a clonogenic assay demonstrating the ability of different combinations of oligonucleotides (1.2, 1.3, and 2.1) to silence KRAS expression, and therefore, affect cell survival of an ovarian cancer cell line containing the LCS6 SNP. Cell survival was normalized to an ovarian cancer cell line that does not contain the LCS6 SNP.
**DETAILED DESCRIPTION**

The invention and disclosures herein are based upon the unexpected discovery that modified or synthetic oligonueleotide (referred to herein as "oligo") molecules restore or improve gene silencing mechanisms or functions that are compromised or lost in cells that cause a variety of disorders, such as cancer. Specifically, modified or synthetic oligo molecules described herein are administered to silence the overexpression of oncogenes. The invention and disclosures are based in part on mutations present within the binding sites, or complementary sites, of wild type miRNAs which modify the binding efficacy of these miRNAs and affect gene silencing. When mutations occur within miRNA binding sites present within proto-oncogenes or oncogenes, the gene silencing functions of at least one wild type miRNA are affected resulting in the overexpression of this proto-oncogene or oncogene. Modified or synthetic oligos described herein are engineered to bind to miRNA binding sites such that gene silencing is either restored, in the case of a mutation which modifies wild type miRNA binding, or enhanced, in the event that a gene is misregulated and overexpressed in the absence of a mutation.

Conveniently, modified or synthetic oligos are engineered to bind to a *let-7* complementary site (LCS) within the KRAS gene or transcript that contains the LCS6 SNP. Subjects who carry the LCS6 SNP display altered expression of this gene compared to subjects who do not carry this mutation. In many subjects who carry the LCS6 SNP, KRAS is overexpressed. KRAS contains eight *let-7* complementary sites (LCSs), however, a single nucleotide polymorphism (SNP) occurring at the 4th position of LCS6 (SEQ ID NO: 21) is predictive of the occurrence of cancer. The presence of the LCS6 SNP modifies the binding efficacy of wild type *let-7* miRNAs. As a result of the LCS6 SNP, KRAS expression is not sufficiently silenced by wild type *let-7* miRNA. The consequence of carrying the LCS6 SNP is the development of cancer.

Accordingly, there is provided compositions and methods for modifying gene expression in subjects who carry mutations in *let-7* complementary sites in order to treat, or alleviate a sign or symptom of, cancer. Alternatively, the subjects are not carriers of such mutations, because genes regulated by *let-7* are up-regulated in many cancer types. The compositions and methods provided herein also provide a therapeutic benefit to these subjects.

For example, using the compositions and methods provided herein, a modified oligo (SEQ ID NO: 22) that has the ability to base-pair with the LCS6 SNP in the KRAS 3'UTR is delivered to human cancer cells that contain the LCS6 SNP. It was demonstrated that this modified oligo (SEQ ID NO: 22) dramatically reduced cell survival in these cells, more so than a naturally occurring miRNA precursor, *let-7*, that is also predicted to target this site (Figure 1). These results demonstrate the unique ability of using these targeting oligonucleotides to reduce growth and/or stimulate death of cancer cells containing the LCS6 SNP. Moreover, the data support using this approach as a therapy in all cancers containing the LCS6 SNP.

### Single Nucleotide Polymorphisms (SNPs)

A single nucleotide polymorphism (SNP) is a DNA sequence variation occurring when a single nucleotide in the genome (or other shared sequence) differs between members of a species (or between paired chromosomes in an individual). SNPs may fall within coding sequences of genes, non-coding regions of genes, or in the intergenic regions between genes. SNPs within a coding sequence will not necessarily change the amino acid sequence of the protein that is produced, due to degeneracy of the genetic code. A SNP mutation that results in a new DNA sequence that encodes the same polypeptide sequence is termed *synonymous* (also referred to as a silent mutation). Conversely, a SNP mutation that results in a new DNA sequence that encodes a different polypeptide sequence is termed *non-synonymous*. SNPs that are not in protein-coding regions may still have consequences for gene splicing, transcription factor binding, or the sequence of non-coding RNA.

SNPs occurring within non-coding regions of genes, e.g. untranslated regions, are particularly important because those regions contain regulatory sequences which are complementary to miRNA molecules and required for interaction with other regulatory factors. SNPs occurring within genomic sequences are transcribed into mRNA transcripts which are targeted by miRNA molecules for degradation or translational silencing. SNPs occurring within the 3' untranslated region (UTR) of the genomic sequence or mRNA transcript of a gene are of particular importance to the methods described herein.

### MicroRNAs

MicroRNAs (miRNAs) are small, non-coding RNAs, recently identified genetic regulators that control cell metabolism, development, cell cycle, cell differentiation and cell death. In addition, miRNAs have been found to be important in cancer, aging, and other disease states, likely due to their ability to regulate hundreds of genes targets.

MiRNAs act by inhibiting translation of messenger RNA (mRNA) into protein by binding to the 3'untranslated region (UTR) of their target mRNAs. It has been found that these microRNA binding sites in 3'UTRs are very highly conserved regions in humans, suggesting an important role in these regions in natural selection. The high degree of conservation of the 3'UTR supports the hypothesis that a disruption of this region leads to disease. While not bound by theory, miRNAs inhibit mRNA translation by either causing mRNA degradation or inhibiting translation itself.

MiRNAs are single-stranded RNA molecules of about 21-23 nucleotides in length. MiRNAs are encoded by endogenous and exogenous genes that are transcribed from DNA by RNA polymerase II, however, miRNA are never translated into polypeptide sequences. As such, miRNA are considered in the art as "non-coding RNA." The term "endogenous" gene as used herein is meant to encompass all genes that naturally occur within the genome of an individual. The term "exogenous" gene as used herein is meant to encompass all genes that do not naturally occur within the genome of an individual. For example, a miRNA could be introduced exogenously by a virus.

While not limited by theory, the present invention and disclosures herein are based in part on the understanding that miRNA biogenesis occurs by the following mechanism. MiRNA are processed from primary mRNA transcripts, called "pri-miRNA" by the nuclease Drosha and the double-stranded RNA binding protein DGCR8/Pasha. Once processed, these transcripts form stem-loop structures referred to as "pre-miRNA". Pre-miRNA are processed one step further by the endonuclease Dicer, which transforms the double-stranded pre-miRNA molecules into the single-stranded mature miRNA and initiates formation of the RNA-induced silencing complex (RISC). One of the two resulting single-stranded complementary miRNA strands, the guide strand, is selected by the argonaute protein of the RISC and incorporated into the RISC, while the other strand, the antiguide or passenger strand, is degraded. Following integration into the RISC, miRNAs bind target miRNAs and subsequently inhibit translation.

MiRNAs are complementary to a part of one or more mRNAs. Moreover, miRNAs
do not require absolute sequence complementarity to bind an mRNA, enabling them to regulate a wide range of target transcripts. As used herein the term "absolute sequence complementarity" is meant to describe a requirement that each nucleotide pair along the length of two sequences, e.g. a miRNA and a target gene or transcript, bind without gaps. It is common that miRNAs bind to their complementary sites with a lesser degree of complementarity. MiRNAs typically bind target sequences with gaps between matched nucleotides. As used herein, the term "complementary" is meant to describe two sequences in which at least 50% of the nucleotides bind from one sequence to the other sequence in trans. MiRNAs have a seed sequence that consists of nucleotides 2-7 or 2-8 at the 5' end of the sequence. The seed sequence binds more closely follows the traditional rules of Watson-Crick base pairing that the 3' sequence of the miRNA. Sequence complementarity between the seed sequence of the miRNA and its target is both necessary and sufficient to determine miRNA binding. Both experimental and computational approaches have determined that complementarity of seven or more base pairs at the miRNA 5' end are sufficient to confer regulation in vivo and are used in biologically relevant targets (Brenneke et al. PLoS Biology. 2005, 3(3): e85). Brenneke et al. also found that extensive complementarity to the 3' sequence of the miRNA is not sufficient to confer regulation without a minimal element of 5' complementarity within the seed sequence. However, the complementarity of the miRNA 3' sequence to its target may determine specificity of miRNAs within a family. Within the seed sequence, G:U base-pairs, single-nucleotide bulges, and mismatches occur which compromise the ability of the miRNA to bind its target. If the seed sequence binding is weakened due to these irregularities, greater complementarity is required within the 3' end of the miRNA to enable efficacious binding of the miRNA to its target. Thus, in order to predict miRNA binding, miRNA sequences and their target sites can be categorized into two groups: those miRNAs for which perfect base pairing occurs at the 5' end and those miRNA for which seed sequence pairing is weaker and the 3' sequence compensates with stronger complementarity. Brenneke et al. refer to these groups as 5' dominant and 3' compensatory, respectively. Exemplary 3' compensatory sites include, but are not limited to, smaller seed matches of 4-6 base-pairs and those seed matches with 7-8 bases and interruptions such as G:U bases, single nucleotide bulges, or mismatches, each of which are accompanied by stronger 3' complementarity.

Modified oligonucleotides described herein are 5' dominant or 3' compensatory. Moreover, in certain circumstances, modified oligonucleotides bind to "canonical" miRNA sites, in which the seed sequence and the 3' sequence of the miRNA both bind strongly to the target sequence. Alternatively, modified oligonucleotides bind to "seed sites" in which the seed sequence of the miRNA alone binds strongly to the target sequence and mediates silencing irrespective of the binding complementarity of the 3' sequence of the miRNA to the target sequence.

MiRNAs are frequently complementary to the 3' UTR of the mRNA transcript. Alternatively, or in addition, miRNA target methylation genomic sites which correspond to genes encoding targeted mRNAs. The methylation state of genomic DNA in part determines the accessibility of that DNA to transcription factors. As such, DNA methylation and de-methylation regulate gene silencing and expression, respectively.

### Oncogenic and Tumor Suppressor MiRNAs

MiRNAs that silence expression of tumor suppressor genes are oncogenes. Alternatively, miRNAs are tumor suppressor genes, which silence the translation of mRNA transcripts of oncogenes. The term "oncogene" as used herein is meant to encompass any gene that, when expressed, directly or indirectly, causes a cell to enter the cell cycle at an inappropriate time or by an uncontrolled mechanism, or fail to die appropriately. Exemplary oncogenes include, but are not limited to, growth factors, transcription factors, regulatory proteins, e.g. GTPases and receptors, and cell cycle proteins. The term "proto-oncogene" as used herein is meant to encompass any gene, which if modified, directly or indirectly, causes a cell to inappropriately enter the cell cycle. Examples of proto-oncogenes include, but are not limited to, RAS, WNT, MYC, ERK and TRK. The term "tumor suppressor gene" as used herein encompasses any gene that repressed or silenced, leads deregulated cell division and/or overexpression of a proto-oncogene or oncogene. Exemplary tumor suppressor genes include, but are not limited to, retinoblastoma (encoding the Rb protein), TP53 (encoding the p53 protein), PTEN, APC, and CD95. Tumor suppressor gene products repress genes that are essential for the continuing of the cell cycle. Effectively, if these genes are not expressed, the cell cycle will not continue, effectively inhibiting cell division. Tumor suppressor gene products couple the cell cycle to DNA damage. Thus, these gene products activate cell cycle checkpoints and DNA repair mechanisms that stall or prevent cell division. If the damage cannot be repaired, the cell initiate apoptosis, or programmed cell death. Some tumor suppressor gene products are involved in cell adhesion, and thus, prevent tumor cells from dispersing, block loss of contact inhibition, and inhibit metastasis. These proteins are also known as metastasis suppressors.

SNPs within the binding site of a tumor suppressing miRNA that decrease binding efficacy, and therefore oncogene silencing, lead to an increased risk, susceptibility or probability of presenting one or more symptoms of a cell proliferative disease. Similarly, SNPs within the binding site of an oncogenic miRNA that increase binding, and therefore increase gene repression, lead to an increased risk, susceptibility or probability of presenting one or more symptoms of a cell proliferative disease.

The disclosure includes all known tumor suppressor and oncogenic miRNAs and their corresponding complementary binding sites. Moreover, all endogenous human miRNAs are encompassed by the disclosure the names, sequences, and targets of which are provided at the database of the Wellcome Trust Sanger Institute MicroRNA Listing for Homo sapiens.

### KRAS Gene

The KRAS gene is one form of RAS in humans. The RAS gene encodes for a protein belongs to a larger superfamily of small GTPases that include the Ras, Rho, Arf, Rab, and Ran families. Functionally, GTPase proteins are molecular switches for a wide variety of signal transduction pathways that control practically every function within a cell. Exemplary functions regulated by GTPase proteins are cytoskeletal integrity, cell proliferation, cell adhesion, apoptosis, and cell migration. Thus, Ras protein deregulated within a cell often leads to increased cell invasion, metastasis, and decreased apoptosis. Importantly, Ras protein is attached to the cell membrane by prenylation and couples growth factor receptors to downstream mitogenic effectors involved in cell proliferation or differentiation.

There are three human RAS genes comprising HRAS, KRAS, and NRAS. Each gene comprises multiple miRNA complementary sites in the 3'UTR of their mRNA transcripts. Specifically, each human RAS gene comprises multiple let-7 complementary sites (LCSs).

Importantly, KRAS is capable of acting as a tumor suppressor gene, a protooncogene, or an oncogene. SNPs in the 3'UTR of KRAS may lead to either increased or decreased binding efficacy of wild type miRNAs. The SNP which occurs in LCS6 (shown below in SEQ ID NOs: 3 and 4), modifies the binding of *let-7* family miRNAs. KRAS acts as a proto-oncogene or oncogene, the LCS6 SNP decreases the binding efficacy of at least one miRNA, causing expressing of the oncogene to be augmented, and the LCS6 SNP is a marker of cell proliferative disease. Alternatively, KRAS acts as a tumor suppressor gene, the LCS6 SNP increases the binding efficacy of at least one miRNAs, causing expression of the tumor suppressor gene to be repressed, and the LCS6 SNP is a marker of cell proliferative disease.

Human KRAS, transcript variant a, is encoded by the following mRNA sequence (NCBI Accession No. NM_033360 and SEQ ID NO: 1) (untranslated regions are bolded, LCS6 is underlined, all sequences provided herein are given from 5' to 3'):

1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gagagtggag gatgcttttt atacattggt
661 gagggagatc cgacaataca gattgaaaaa aatcagcaaa gaagaaaaga ctcctggctg
721 tgtgaaaatt aaaaaatgca ttataatgta atctgggtgt tgatgatgcc ttctatacat
781 tagttcgaga aattcgaaaa cataaagaaa agatgagcaa agatggtaaa aagaagaaaa
841 agaagtcaaa gacaaagtgt gtaattatgt aaatacaatt tgtacttttt tcttaaggca
901 tactagtaca agtggtaatt tttgtacatt acactaaatt attagcattt gttttagcat
961 tacctaattt ttttcctgct ccatgcagac tgttagcttt taccttaaat gcttatttta
1021 aaatgacagt ggaagttttt ttttcctcta agtgccagta ttcccagagt tttggttttt
1081 gaactagcaa tgcctgtgaa aaagaaactg aatacctaag atttctgtct tggggttttt
1141 ggtgcatgca gttgattact tcttattttt cttaccaatt gtgaatgttg gtgtgaaaca
1201 aattaatgaa gcttttgaat catccctatt ctgtgtttta tctagtcaca taaatggatt
1261 aattactaat ttcagttgag accttctaat tggtttttac tgaaacattg agggaacaca
1321 aatttatggg cttcctgatg atgattcttc taggcatcat gtcctatagt ttgtcatccc
1381 tgatgaatgt aaagttacac tgttcacaaa ggttttgtct cctttccact gctattagtc
1441 atggtcactc tccccaaaat attatatttt ttctataaaa agaaaaaaat ggaaaaaaat
1501 tacaaggcaa tggaaactat tataaggcca tttccttttc acattagata aattactata
1561 aagactccta atagcttttc ctgttaaggc agacccagta tgaaatgggg attattatag
1621 caaccatttt ggggctatat ttacatgcta ctaaattttt ataataattg aaaagatttt
1681 aacaagtata aaaaattctc ataggaatta aatgtagtct ccctgtgtca gactgctctt
1741 tcatagtata actttaaatc ttttcttcaa cttgagtctt tgaagatagt tttaattctg
1801 cttgtgacat taaaagatta tttgggccag ttatagctta ttaggtgttg aagagaccaa
1861 ggttgcaagg ccaggccctg tgtgaacctt tgagctttca tagagagttt cacagcatgg
1921 actgtgtccc cacggtcatc cagtgttgtc atgcattggt tagtcaaaat ggggagggac
1981 tagggcagtt tggatagctc aacaagatac aatctcactc tgtggtggtc ctgctgacaa
2041 atcaagagca ttgcttttgt ttcttaagaa aacaaactct tttttaaaaa ttacttttaa
2101 atattaactc aaaagttgag attttggggt ggtggtgtgc caagacatta attttttttt
2161 taaacaatga agtgaaaaag ttttacaatc tctaggtttg gctagttctc ttaacactgg
2221 ttaaattaac attgcataaa cacttttcaa gtctgatcca tatttaataa tgctttaaaa

2281 taaaaataaa aacaatcctt ttgataaatt taaaatgtta cttattttaa aataaatgaa
2341 gtgagatggc atggtgaggt gaaagtatca ctggactagg aagaaggtga cttaggttct
2401 agataggtgt cttttaggac tctgattttg aggacatcac ttactatcca tttcttcatg
2461 ttaaaagaag tcatctcaaa ctcttagttt ttttttttta caactatgta atttatattc
2521 catttacata aggatacact tatttgtcaa gctcagcaca atctgtaaat ttttaaccta
2581 tgttacacca tcttcagtgc cagtcttggg caaaattgtg caagaggtga agtttatatt
2641 tgaatatcca ttctcgtttt aggactcttc ttccatatta gtgtcatctt gcctccctac
2701 cttccacatg ccccatgact tgatgcagtt ttaatacttg taattcccct aaccataaga
2761 tttactgctg ctgtggatat ctccatgaag ttttcccact gagtcacatc agaaatgccc
2821 tacatcttat ttcctcaggg ctcaagagaa tctgacagat accataaagg gatttgacct
2881 aatcactaat tttcaggtgg tggctgatgc tttgaacatc tctttgctgc ccaatccatt
2941 agcgacagta ggatttttca aacctggtat gaatagacag aaccctatcc agtggaagga
3001 gaatttaata aagatagtgc tgaaagaatt ccttaggtaa tctataacta ggactactcc
3061 tggtaacagt aatacattcc attgttttag taaccagaaa tcttcatgca atgaaaaata
3121 ctttaattca tgaagcttac tttttttttt tggtgtcaga gtctcgctct tgtcacccag
3181 gctggaatgc agtggcgcca tctcagctca ctgcaacctc catctcccag gttcaagcga
3241 ttctcgtgcc tcggcctcct gagtagctgg gattacaggc gtgtgccact acactcaact
3301 aatttttgta tttttaggag agacggggtt tcaccctgtt ggccaggctg gtctcgaact
3361 cctgacctca agtgattcac ccaccttggc ctcataaacc tgttttgcag aactcattta
3421 ttcagcaaat atttattgag tgcctaccag atgccagtca ccgcacaagg cactgggtat
3481 atggtatccc caaacaagag acataatccc ggtccttagg tagtgctagt gtggtctgta
3541 atatcttact aaggcctttg gtatacgacc cagagataac acgatgcgta ttttagtttt
3601 gcaaagaagg ggtttggtct ctgtgccagc tctataattg ttttgctacg attccactga
3661 aactcttcga tcaagctact ttatgtaaat cacttcattg ttttaaagga ataaacttga
3721 ttatattgtt tttttatttg gcataactgt gattctttta ggacaattac tgtacacatt
3781 aaggtgtatg tcagatattc atattgaccc aaatgtgtaa tattccagtt ttctctgcat
3841 aagtaattaa aatatactta aaaattaata gttttatctg ggtacaaata aacaggtgcc
3901 tgaactagtt cacagacaag gaaacttcta tgtaaaaatc actatgattt ctgaattgct
3961 atgtgaaact acagatcttt ggaacactgt ttaggtaggg tgttaagact tacacagtac
4021 ctcgtttcta cacagagaaa gaaatggcca tacttcagga actgcagtgc ttatgagggg
4081 atatttaggc ctcttgaatt tttgatgtag atgggcattt ttttaaggta gtggttaatt
4141 acctttatgt gaactttgaa tggtttaaca aaagatttgt ttttgtagag attttaaagg
4201 gggagaattc tagaaataaa tgttacctaa ttattacagc cttaaagaca aaaatccttg
4261 ttgaagtttt tttaaaaaaa gctaaattac atagacttag gcattaacat gtttgtggaa
4321 gaatatagca gacgtatatt gtatcatttg agtgaatgtt cccaagtagg cattctaggc
4381 tctatttaac tgagtcacac tgcataggaa tttagaacct aacttttata ggttatcaaa
4441 actgttgtca ccattgcaca attttgtcct aatatataca tagaaacttt gtggggcatg
4501 ttaagttaca gtttgcacaa gttcatctca tttgtattcc attgattttt tttttcttct
4561 aaacattttt tcttcaaaca gtatataact tttttagggg gatttttttt tagacagcaa
4621 aaactatctg aagatttcca tttgtcaaaa agtaatgatt tcttgataat tgtgtagtaa
4681 tgttttttag aacccagcag ttaccttaaa gctgaattta tatttagtaa cttctgtgtt
4741 aatactggat agcatgaatt ctgcattgag aaactgaata gctgtcataa aatgaaactt
4801 tctttctaaa gaaagatact cacatgagtt cttgaagaat agtcataact agattaagat
4861 ctgtgtttta gtttaatagt ttgaagtgcc tgtttgggat aatgataggt aatttagatg
4921 aatttagggg aaaaaaaagt tatctgcaga tatgttgagg gcccatctct cccccacac
4981 ccccacagag ctaactgggt tacagtgttt tatccgaaag tttccaattc cactgtcttg
5041 tgttttcatg ttgaaaatac ttttgcattt ttcctttgag tgccaatttc ttactagtac
5101 tatttcttaa tgtaacatgt ttacctggaa tgtattttaa ctatttttgt atagtgtaaa
5161 ctgaaacatg cacattttgt acattgtgct ttcttttgtg ggacatatgc agtgtgatcc
5221 agttgttttc catcatttgg ttgcgctgac ctaggaatgt tggtcatatc aaacattaaa
5281 aatgaccact cttttaattg aaattaactt ttaaatgttt ataggagtat gtgctgtgaa
5341 gtgatctaaa atttgtaata tttttgtcat gaactgtact actcctaatt attgtaatgt
5401 aataaaaata gttacagtga caaaaaaaaa aaaaaa

Human KRAS, transcript variant b, is encoded by the following mRNA sequence (NCBI Accession No. NM 004985 and SEQ ID NO: 2)(untranslated regions are bolded, LCS6 is underlined):

1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gggtgttgat gatgccttct atacattagt
661 tcgagaaatt cgaaaacata aagaaaagat gagcaaagat ggtaaaaaga agaaaaagaa
721 gtcaaagaca aagtgtgtaa ttatgtaaat acaatttgta cttttttctt aaggcatact
781 agtacaagtg gtaatttttg tacattacac taaattatta gcatttgttt tagcattacc
841 taattttttt cctgctccat gcagactgtt agcttttacc ttaaatgctt attttaaaat
901 gacagtggaa gttttttttt cctctaagtg ccagtattcc cagagttttg gtttttgaac
961 tagcaatgcc tgtgaaaaag aaactgaata cctaagattt ctgtcttggg gtttttggtg
1021 catgcagttg attacttctt atttttctta ccaattgtga atgttggtgt gaaacaaatt
1081 aatgaagctt ttgaatcatc cctattctgt gttttatcta gtcacataaa tggattaatt
1141 actaatttca gttgagacct tctaattggt ttttactgaa acattgaggg aacacaaatt
1201 tatgggcttc ctgatgatga ttcttctagg catcatgtcc tatagtttgt catccctgat
1261 gaatgtaaag ttacactgtt cacaaaggtt ttgtctcctt tccactgcta ttagtcatgg
1321 tcactctccc caaaatatta tattttttct ataaaaagaa aaaaatggaa aaaaattaca
1381 aggcaatgga aactattata aggccatttc cttttcacat tagataaatt actataaaga
1441 ctcctaatag cttttcctgt taaggcagac ccagtatgaa atggggatta ttatagcaac
1501 cattttgggg ctatatttac atgctactaa atttttataa taattgaaaa gattttaaca
1561 agtataaaaa attctcatag gaattaaatg tagtctccct gtgtcagact gctctttcat
1621 agtataactt taaatctttt cttcaacttg agtctttgaa gatagtttta attctgcttg
1681 tgacattaaa agattatttg ggccagttat agcttattag gtgttgaaga gaccaaggtt
1741 gcaaggccag gccctgtgtg aacctttgag ctttcataga gagtttcaca gcatggactg
1801 tgtccccacg gtcatccagt gttgtcatgc attggttagt caaaatgggg agggactagg
1861 gcagtttgga tagctcaaca agatacaatc tcactctgtg gtggtcctgc tgacaaatca
1921 agagcattgc ttttgtttct taagaaaaca aactcttttt taaaaattac ttttaaatat
1981 taactcaaaa gttgagattt tggggtggtg gtgtgccaag acattaattt tttttttaaa
2041 caatgaagtg aaaaagtttt acaatctcta ggtttggcta gttctcttaa cactggttaa
2101 attaacattg cataaacact tttcaagtct gatccatatt taataatgct ttaaaataaa
2161 aataaaaaca atccttttga taaatttaaa atgttactta ttttaaaata aatgaagtga
2221 gatggcatgg tgaggtgaaa gtatcactgg actaggaaga aggtgactta ggttctagat
2281 aggtgtcttt taggactctg attttgagga catcacttac tatccatttc ttcatgttaa
2341 aagaagtcat ctcaaactct tagttttttt tttttacaac tatgtaattt atattccatt
2401 tacataagga tacacttatt tgtcaagctc agcacaatct gtaaattttt aacctatgtt
2461 acaccatctt cagtgccagt cttgggcaaa attgtgcaag aggtgaagtt tatatttgaa
2521 tatccattct cgttttagga ctcttcttcc atattagtgt catcttgcct ccctaccttc
2581 cacatgcccc atgacttgat gcagttttaa tacttgtaat tcccctaacc ataagattta
2641 ctgctgctgt ggatatctcc atgaagtttt cccactgagt cacatcagaa atgccctaca
2701 tcttatttcc tcagggctca agagaatctg acagatacca taaaggattt gacctaatc
2761 actaattttc aggtggtggc tgatgctttg aacatctctt tgctgcccaa tccattagcg
2821 acagtaggat ttttcaaacc tggtatgaat agacagaacc ctatccagtg gaaggagaat
2881 ttaataaaga tagtgctgaa agaattcctt aggtaatcta taactaggac tactcctggt
2941 aacagtaata cattccattg ttttagtaac cagaaatctt catgcaatga aaaatacttt
3001 aattcatgaa gcttactttt tttttttggt gtcagagtct cgctcttgtc acccaggctg
3061 gaatgcagtg gcgccatctc agctcactgc aacctccatc tcccaggttc aagcgattct
3121 cgtgcctcgg cctcctgagt agctgggatt acaggcgtgt gccactacac tcaactaatt
3181 tttgtatttt taggagagac ggggtttcac cctgttggcc aggctggtct cgaactcctg
3241 acctcaagtg attcacccac cttggcctca taaacctgtt ttgcagaact catttattca
3301 gcaaatattt attgagtgcc taccagatgc cagtcaccgc acaaggcact gggtatatgg

12

3361 tatccccaaa caagagacat aatcccggtc cttaggtagt gctagtgtgg tctgtaatat
3421 cttactaagg cctttggtat acgacccaga gataacacga tgcgtatttt agttttgcaa
3481 agaaggggtt tggtctctgt gccagctcta taattgtttt gctacgattc cactgaaact
3541 cttcgatcaa gctactttat gtaaatcact tcattgtttt aaaggaataa acttgattat
3601 attgtttttt tatttggcat aactgtgatt cttttaggac aattactgta cacattaagg
3661 tgtatgtcag atattcatat tgacccaaat gtgtaatatt ccagttttct ctgcataagt
3721 aattaaaata tacttaaaaa ttaatagttt tatctgggta caaataaaca ggtgcctgaa
3781 ctagttcaca gacaaggaaa cttctatgta aaaatcacta tgatttctga attgctatgt
3841 gaaactacag atctttggaa cactgtttag gtagggtgtt aagacttaca cagtacctcg
3901 tttctacaca gagaaagaaa tggccatact tcaggaactg cagtgcttat gaggggatat
3961 ttaggcctct tgaatttttg atgtagatgg gcattttttt aaggtagtgg ttaattacct
4021 ttatgtgaac tttgaatggt ttaacaaaag atttgttttt gtagagattt taaaggggga
4081 gaattctaga aataaatgtt acctaattat tacagcctta aagacaaaaa tccttgttga
4141 agttttttta aaaaaagcta aattacatag acttaggcat taacatgttt gtggaagaat
4201 atagcagacg tatattgtat catttgagtg aatgttccca agtaggcatt ctaggctcta
4261 tttaactgag tcacactgca taggaattta gaacctaact tttataggtt atcaaaactg
4321 ttgtcaccat tgcacaattt tgtcctaata tatacataga aactttgtgg ggcatgttaa
4381 gttacagttt gcacaagttc atctcatttg tattccattg attttttttt tcttctaaac
4441 atttttcttt caaacagtat ataacttttt ttaggggatt tttttttaga cagcaaaaac
4501 tatctgaaga tttccatttg tcaaaaagta atgatttctt gataattgtg tagtaatgtt
4561 ttttagaacc cagcagttac cttaaagctg aatttatatt tagtaacttc tgtgttaata
4621 ctggatagca tgaattctgc attgagaaac tgaatagctg tcataaaatg aaactttctt
4681 tctaaagaaa gatactcaca tgagttcttg aagaatagtc ataactagat taagatctgt
4741 gttttagttt aatagtttga agtgcctgtt tgggataatg ataggtaatt tagatgaatt
4801 taggggaaaa aaaagttatc tgcagatatg ttgagggccc atctctcccc ccacaccccc
4861 acagagctaa ctgggttaca gtgttttatc cgaaagtttc caattccact gtcttgtgtt
4921 ttcatgttga aaatactttt gcatttttcc tttgagtgcc aatttcttac tagtactatt
4981 tcttaatgta acatgtttac ctggaatgta ttttaactat ttttgtatag tgtaaactga
5041 aacatgcaca ttttgtacat tgtgctttct tttgtgggac atatgcagtg tgatccagtt
5101 gttttccatc atttggttgc gctgacctag gaatgttggt catatcaaac attaaaaatg
5161 accactcttt taattgaaat taacttttaa atgtttatag gagtatgtgc tgtgaagtga
5221 tctaaaattt gtaatatttt tgtcatgaac tgtactactc ctaattattg taatgtaata
5281 aaaatagtta cagtgacaaa aaaaaaaaaa aa

Human KRAS, transcript variant a, comprising the LCS6 SNP, is encoded by the following mRNA sequence (SEQ ID NO: 3) (untranslated regions are bolded, LCS6 is underlined, SNP is capitalized):

1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gagagtggag gatgcttttt atacattggt
661 gagggagatc cgacaataca gattgaaaaa aatcagcaaa gaagaaaaga ctcctggctg
721 tgtgaaaatt aaaaaatgca ttataatgta atctgggtgt tgatgatgcc ttctatacat
781 tagttcgaga aattcgaaaa cataaagaaa agatgagcaa agatggtaaa aagaagaaaa
841 agaagtcaaa gacaaagtgt gtaattatgt aaatacaatt tgtacttttt tcttaaggca
901 tactagtaca agtggtaatt tttgtacatt acactaaatt attagcattt gttttagcat
961 tacctaattt ttttcctgct ccatgcagac tgttagcttt taccttaaat gcttatttta
1021 aaatgacagt ggaagttttt ttttcctcta agtgccagta ttcccagagt tttggttttt
1081 gaactagcaa tgcctgtgaa aaagaaactg aatacctaag atttctgtct tggggttttt

1141 ggtgcatgca gttgattact tcttattttt cttaccaatt gtgaatgttg gtgtgaaaca
1201 aattaatgaa gcttttgaat catccctatt ctgtgtttta tctagtcaca taaatggatt
1261 aattactaat ttcagttgag accttctaat tggtttttac tgaaacattg agggaacaca
1321 aatttatggg cttcctgatg atgattcttc taggcatcat gtcctatagt ttgtcatccc
1381 tgatgaatgt aaagttacac tgttcacaaa ggttttgtct cctttccact gctattagtc
1441 atggtcactc tccccaaaat attatatttt ttctataaaa agaaaaaaat ggaaaaaaat
1501 tacaaggcaa tggaaactat tataaggcca tttccttttc acattagata aattactata
1561 aagactccta atagcttttc ctgttaaggc agacccagta tgaaatgggg attattatag
1621 caaccatttt ggggctatat ttacatgcta ctaaattttt ataataattg aaaagatttt
1681 aacaagtata aaaaattctc ataggaatta aatgtagtct ccctgtgtca gactgctctt
1741 tcatagtata actttaaatc ttttcttcaa cttgagtctt tgaagatagt tttaattctg
1801 cttgtgacat taaaagatta tttgggccag ttatagctta ttaggtgttg aagagaccaa
1861 ggttgcaagg ccaggccctg tgtgaacctt tgagctttca tagagagttt cacagcatgg
1921 actgtgtccc cacggtcatc cagtgttgtc atgcattggt tagtcaaaat ggggagggac
1981 tagggcagtt tggatagctc aacaagatac aatctcactc tgtggtggtc ctgctgacaa
2041 atcaagagca ttgcttttgt ttcttaagaa aacaaactct tttttaaaaa ttacttttaa
2101 atattaactc aaaagttgag attttggggt ggtggtgtgc caagacatta attttttttt
2161 taaacaatga agtgaaaaag ttttacaatc tctaggtttg gctagttctc ttaacactgg
2221 ttaaattaac attgcataaa cacttttcaa gtctgatcca tatttaataa tgctttaaaa
2281 taaaaataaa aacaatcctt ttgataaatt taaaatgtta cttattttaa aataaatgaa
2341 gtgagatggc atggtgaggt gaaagtatca ctggactagg aagaaggtga cttaggttct
2401 agataggtgt cttttaggac tctgattttg aggacatcac ttactatcca tttcttcatg
2461 ttaaaagaag tcatctcaaa ctcttagttt ttttttttta caactatgta atttatattc
2521 catttacata aggatacact tatttgtcaa gctcagcaca atctgtaaat ttttaaccta
2581 tgttacacca tcttcagtgc cagtcttggg caaaattgtg caagaggtga agtttatatt
2641 tgaatatcca ttctcgtttt aggactcttc ttccatatta gtgtcatctt gcctccctac
2701 cttccacatg ccccatgact tgatgcagtt ttaatacttg taattcccct aaccataaga
2761 tttactgctg ctgtggatat ctccatgaag ttttcccact gagtcacatc agaaatgccc
2821 tacatcttat ttcctcaggg ctcaagagaa tctgacagat accataaagg gatttgacct
2881 aatcactaat tttcaggtgg tggctgatgc tttgaacatc tctttgctgc ccaatccatt
2941 agcgacagta ggatttttca aacctggtat gaatagacag aaccctatcc agtggaagga
3001 gaatttaata aagatagtgc tgaaagaatt ccttaggtaa tctataacta ggactactcc
3061 tggtaacagt aatacattcc attgttttag taaccagaaa tcttcatgca atgaaaaata
3121 ctttaattca tgaagcttac tttttttttt tggtgtcaga gtctcgctct tgtcacccag
3181 gctggaatgc agtggcgcca tctcagctca ctgcaacctc catctcccag gttcaagcga
3241 ttctcgtgcc tcggcctcct gagtagctgg gattacaggc gtgtgccact acactcaact
3301 aatttttgta tttttaggag agacggggtt tcaccctgtt ggccaggctg gtctcgaact
3361 cctgacctca agtgatGcac ccaccttggc ctcataaacc tgttttgcag aactcattta
3421 ttcagcaaat atttattgag tgcctaccag atgccagtca ccgcacaagg cactgggtat
3481 atggtatccc caaacaagag acataatccc ggtccttagg tagtgctagt gtggtctgta
3541 atatcttact aaggcctttg gtatacgacc cagagataac acgatgcgta ttttagtttt
3601 gcaaagaagg ggtttggtct ctgtgccagc tctataattg ttttgctacg attccactga
3661 aactcttcga tcaagctact ttatgtaaat cacttcattg ttttaaagga ataaacttga
3721 ttatattgtt tttttatttg gcataactgt gattctttta ggacaattac tgtacacatt
3781 aaggtgtatg tcagatattc atattgaccc aaatgtgtaa tattccagtt ttctctgcat
3841 aagtaattaa aatatactta aaaattaata gttttatctg ggtacaaata aacaggtgcc
3901 tgaactagtt cacagacaag gaaacttcta tgtaaaaatc actatgattt ctgaattgct
3961 atgtgaaact acagatcttt ggaacactgt ttaggtaggg tgttaagact tacacagtac
4021 ctcgtttcta cacagagaaa gaaatggcca tacttcagga actgcagtgc ttatgagggg
4081 atatttaggc ctcttgaatt tttgatgtag atgggcattt ttttaaggta gtggttaatt
4141 acctttatgt gaactttgaa tggtttaaca aaagatttgt ttttgtagag attttaaagg
4201 gggagaattc tagaaataaa tgttacctaa ttattacagc cttaaagaca aaaatccttg
4261 ttgaagtttt tttaaaaaaa gctaaattac atagacttag gcattaacat gtttgtggaa
4321 gaatatagca gacgtatatt gtatcatttg agtgaatgtt cccaagtagg cattctaggc
4381 tctatttaac tgagtcacac tgcataggaa tttagaacct aacttttata ggttatcaaa
4441 actgttgtca ccattgcaca attttgtcct aatatataca tagaaacttt gtggggcatg
4501 ttaagttaca gtttgcacaa gttcatctca tttgtattcc attgattttt tttttcttct
4561 aaacattttt tcttcaaaca gtatataact tttttagggg gatttttttt tagacagcaa

4621 aaactatctg aagatttcca tttgtcaaaa agtaatgatt tcttgataat tgtgtagtaa
4681 tgttttttag aacccagcag ttaccttaaa gctgaattta tatttagtaa cttctgtgtt
4741 aatactggat agcatgaatt ctgcattgag aaactgaata gctgtcataa aatgaaactt
4801 tctttctaaa gaaagatact cacatgagtt cttgaagaat agtcataact agattaagat
4861 ctgtgtttta gtttaatagt ttgaagtgcc tgtttgggat aatgataggt aatttagatg
4921 aatttagggg aaaaaaaagt tatctgcaga tatgttgagg gcccatctct ccccccacac
4981 ccccacagag ctaactgggt tacagtgttt tatccgaaag tttccaattc cactgtcttg
5041 tgttttcatg ttgaaaatac ttttgcattt ttcctttgag tgccaatttc ttactagtac
5101 tatttcttaa tgtaacatgt ttacctggaa tgtattttaa ctatttttgt atagtgtaaa
5161 ctgaaacatg cacattttgt acattgtgct ttcttttgtg ggacatatgc agtgtgatcc
5221 agttgttttc catcatttgg ttgcgctgac ctaggaatgt tggtcatatc aaacattaaa
5281 aatgaccact cttttaattg aaattaactt ttaaatgttt ataggagtat gtgctgtgaa
5341 gtgatctaaa atttgtaata tttttgtcat gaactgtact actcctaatt attgtaatgt
5401 aataaaaata gttacagtga caaaaaaaaa aaaaaa

Human KRAS, transcript variant b, comprising the LCS6 SNP, is encoded by the following mRNA sequence (SEQ ID NO: 4)(untranslated regions are bolded, LCS6 is underlined, SNP is capitalized):

1 ggccgcggcg gcggaggcag cagcggcggc ggcagtggcg gcggcgaagg tggcggcggc
61 tcggccagta ctcccggccc ccgccatttc ggactgggag cgagcgcggc gcaggcactg
121 aaggcggcgg cggggccaga ggctcagcgg ctcccaggtg cgggagagag gcctgctgaa
181 aatgactgaa tataaacttg tggtagttgg agctggtggc gtaggcaaga gtgccttgac
241 gatacagcta attcagaatc attttgtgga cgaatatgat ccaacaatag aggattccta
301 caggaagcaa gtagtaattg atggagaaac ctgtctcttg gatattctcg acacagcagg
361 tcaagaggag tacagtgcaa tgagggacca gtacatgagg actggggagg gctttctttg
421 tgtatttgcc ataaataata ctaaatcatt tgaagatatt caccattata gagaacaaat
481 taaaagagtt aaggactctg aagatgtacc tatggtccta gtaggaaata aatgtgattt
541 gccttctaga acagtagaca caaaacaggc tcaggactta gcaagaagtt atggaattcc
601 ttttattgaa acatcagcaa agacaagaca gggtgttgat gatgccttct atacattagt
661 tcgagaaatt cgaaaacata aagaaaagat gagcaaagat ggtaaaaaga agaaaaagaa
721 gtcaaagaca aagtgtgtaa ttatgtaaat acaatttgta cttttttctt aaggcatact
781 agtacaagtg gtaatttttg tacattacac taaattatta gcatttgttt tagcattacc
841 taattttttt cctgctccat gcagactgtt agcttttacc ttaaatgctt attttaaaat
901 gacagtggaa gttttttttt cctctaagtg ccagtattcc cagagttttg gtttttgaac
961 tagcaatgcc tgtgaaaaag aaactgaata cctaagattt ctgtcttggg gtttttggtg
1021 catgcagttg attacttctt atttttctta ccaattgtga atgttggtgt gaaacaaatt
1081 aatgaagctt ttgaatcatc cctattctgt gttttatcta gtcacataaa tggattaatt
1141 actaatttca gttgagacct tctaattggt ttttactgaa acattgaggg aacacaaatt
1201 tatgggcttc ctgatgatga ttcttctagg catcatgtcc tatagtttgt catccctgat
1261 gaatgtaaag ttacactgtt cacaaaggtt ttgtctcctt tccactgcta ttagtcatgg
1321 tcactctccc caaaatatta tattttttct ataaaaagaa aaaaatggaa aaaaattaca
1381 aggcaatgga aactattata aggccatttc cttttcacat tagataaatt actataaaga
1441 ctcctaatag cttttcctgt taaggcagac ccagtatgaa atggggatta ttatagcaac
1501 cattttgggg ctatatttac atgctactaa atttttataa taattgaaaa gattttaaca
1561 agtataaaaa attctcatag gaattaaatg tagtctccct gtgtcagact gctctttcat
1621 agtataactt taaatctttt cttcaacttg agtctttgaa gatagtttta attctgcttg
1681 tgacattaaa agattatttg ggccagttat agcttattag gtgttgaaga gaccaaggtt
1741 gcaaggccag gccctgtgtg aacctttgag ctttcataga gagtttcaca gcatggactg
1801 tgtccccacg gtcatccagt gttgtcatgc attggttagt caaaatgggg agggactagg
1861 gcagtttgga tagctcaaca agatacaatc tcactctgtg gtggtcctgc tgacaaatca
1921 agagcattgc ttttgtttct taagaaaaca aactcttttt taaaaattac ttttaaatat
1981 taactcaaaa gttgagattt tggggtggtg gtgtgccaag acattaattt tttttttaaa
2041 caatgaagtg aaaaagtttt acaatctcta ggtttggcta gttctcttaa cactggttaa
2101 attaacattg cataaacact tttcaagtct gatccatatt taataatgct ttaaaataaa
2161 aataaaaaca atccttttga taaatttaaa atgttactta ttttaaaata aatgaagtga

2221 gatggcatgg tgaggtgaaa gtatcactgg actaggaaga aggtgactta ggttctagat
2281 aggtgtcttt taggactctg attttgagga catcacttac tatccatttc ttcatgttaa
2341 aagaagtcat ctcaaactct tagttttttt tttttacaac tatgtaattt atattccatt
2401 tacataagga tacacttatt tgtcaagctc agcacaatct gtaaattttt aacctatgtt
2461 acaccatctt cagtgccagt cttgggcaaa attgtgcaag aggtgaagtt tatatttgaa
2521 tatccattct cgttttagga ctcttcttcc atattagtgt catcttgcct ccctaccttc
2581 cacatgcccc atgacttgat gcagttttaa tacttgtaat tcccctaacc ataagattta
2641 ctgctgctgt ggatatctcc atgaagtttt cccactgagt cacatcagaa atgccctaca
2701 tcttatttcc tcaggctcaa agagaatctg acagatacca taaaggattt tgacctaatc
2761 actaattttc aggtggtggc tgatgctttg aacatctctt tgctgcccaa tccattagcg
2821 acagtaggat ttttcaaacc tggtatgaat agacagaacc ctatccagtg gaaggagaat
2881 ttaataaaga tagtgctgaa agaattcctt aggtaatcta taactaggac tactcctggt
2941 aacagtaata cattccattg ttttagtaac cagaaatctt catgcaatga aaaatacttt
3001 aattcatgaa gcttactttt tttttttggt gtcagagtct cgctcttgtc acccaggctg
3061 gaatgcagtg gcgccatctc agctcactgc aacctccatc tcccaggttc aagcgattct
3121 cgtgcctcgg cctcctgagt agctgggatt acaggcgtgt gccactacac tcaactaatt
3181 tttgtatttt taggagagac ggggtttcac cctgttggcc aggctggtct cgaactcctg
3241 acctcaagtg atGcacccac cttggcctca taaacctgtt ttgcagaact catttattca
3301 gcaaatattt attgagtgcc taccagatgc cagtcaccgc acaaggcact gggtatatgg
3361 tatccccaaa caagagacat aatcccggtc cttaggtagt gctagtgtgg tctgtaatat
3421 cttactaagg cctttggtat acgacccaga gataacacga tgcgtatttt agttttgcaa
3481 agaaggggtt tggtctctgt gccagctcta taattgtttt gctacgattc cactgaaact
3541 cttcgatcaa gctactttat gtaaatcact tcattgtttt aaaggaataa acttgattat
3601 attgtttttt tatttggcat aactgtgatt cttttaggac aattactgta cacattaagg
3661 tgtatgtcag atattcatat tgacccaaat gtgtaatatt ccagttttct ctgcataagt
3721 aattaaaata tacttaaaaa ttaatagttt tatctgggta caaataaaca ggtgcctgaa
3781 ctagttcaca gacaaggaaa cttctatgta aaaatcacta tgatttctga attgctatgt
3841 gaaactacag atctttggaa cactgtttag gtagggtgtt aagacttaca cagtacctcg
3901 tttctacaca gagaaagaaa tggccatact tcaggaactg cagtgcttat gaggggatat
3961 ttaggcctct tgaatttttg atgtagatgg gcattttttt aaggtagtgg ttaattacct
4021 ttatgtgaac tttgaatggt ttaacaaaag atttgttttt gtagagattt taaaggggga
4081 gaattctaga aataaatgtt acctaattat tacagcctta aagacaaaaa tccttgttga
4141 agttttttta aaaaaagcta aattacatag acttaggcat taacatgttt gtggaagaat
4201 atagcagacg tatattgtat catttgagtg aatgttccca agtaggcatt ctaggctcta
4261 tttaactgag tcacactgca taggaattta gaacctaact tttataggtt atcaaaactg
4321 ttgtcaccat tgcacaattt tgtcctaata tatacataga aactttgtgg ggcatgttaa
4381 gttacagttt gcacaagttc atctcatttg tattccattg attttttttt tcttctaaac
4441 atttttcttt caaacagtat ataacttttt ttaggggatt tttttttaga cagcaaaaac
4501 tatctgaaga tttccatttg tcaaaaagta atgatttctt gataattgtg tagtaatgtt
4561 ttttagaacc cagcagttac cttaaagctg aatttatatt tagtaacttc tgtgttaata
4621 ctggatagca tgaattctgc attgagaaac tgaatagctg tcataaaatg aaactttctt
4681 tctaaagaaa gatactcaca tgagttcttg aagaatagtc ataactagat taagatctgt
4741 gttttagttt aatagtttga agtgcctgtt tgggataatg ataggtaatt tagatgaatt
4801 taggggaaaa aaaagttatc tgcagatatg ttgagggccc atctctcccc ccacaccccc
4861 acagagctaa ctgggttaca gtgttttatc cgaaagtttc caattccact gtcttgtgtt
4921 ttcatgttga aaatactttt gcatttttcc tttgagtgcc aatttcttac tagtactatt
4981 tcttaatgta acatgtttac ctggaatgta ttttaactat ttttgtatag tgtaaactga
5041 aacatgcaca ttttgtacat tgtgctttct tttgtgggac atatgcagtg tgatccagtt
5101 gttttccatc atttggttgc gctgacctag gaatgttggt catatcaaac attaaaaatg
5161 accactcttt taattgaaat taacttttaa atgtttatag gagtatgtgc tgtgaagtga
5221 tctaaaattt gtaatatttt tgtcatgaac tgtactactc ctaattattg taatgtaata
5281 aaaatagtta cagtgacaaa aaaaaaaaaa aa

### Modified Oligonucleotides

As used herein, the term "let-7 complementary site" is meant to describe any region of a gene or gene transcript that binds a member of the *let-7* family of miRNAs. Moreover, this term encompasses those sequences within a gene or gene transcript that are complementary to the sequence of a *left-7* family miRNAs.

The Human KRAS 3' UTR comprises 8 LCSs named LCS1-LCS8, respectively. For the following sequences, thymine (T) may be substituted for uracil (U). LCS1 comprises the sequence GACAGUGGAAGUUUUUUUUUCGUCG (SEQ ID NO: 5). LCS2 comprises the sequence AUUAGUGUCAUCUUGCCUC (SEQ ID NO: 6). LCS3 comprises the sequence AAUGCCCUACAUCUUAUUUUCCUCA (SEQ ID NO: 7). LCS4 comprises the sequence GGUUCAAGCGAUUCUCGUGCCUCG (SEQ ID NO: 8). LCS5 comprises the sequence GGGUGGUCGGAACUCCUGACCUCA (SEQ ID NO: 9). LCS6 comprises the sequence GAUUCACCCACCUUGGCCUCA (SEQ ID NO: 10). LCS7 comprises the sequence GGGUGUUAAGACUUGACACAGUACCUCG (SEQ ID NO: 11). LCS8 comprises the sequence AGUGCUUAUGAGGGGAUAUUUAGGCCUC (SEQ ID NO: 12).

There is herein provided compositions containing modified oligos that bind to the LCS6 SNP defined by the sequence GAUGCACCCACCUUGGCCUCA (SNP bolded for emphasis) (SEQ ID NO: 13). Preferably, the modified oligo is derived from *let-7b*. For the preceding sequence, thymine (T) may be substituted for uracil (U).

There is herein provided compositions comprising modified oligos that are derived from wild type *let-7* family miRNAs. Exemplary *let-7* miRNAs include, but are not limited to, *let-7a*, *let-7b*, *let-7c*, *let-7d*, *let-7e*, *let-7f*, *let-7g*, *let-7h*, and *let-7i*. For the following sequences, thymine (T) may be substituted for uracil (U). *let-7a* comprises the sequence UUGAUAUGUUGGAUGAUGGAGU (SEQ ID NO: 14). *let-7b* comprises the sequence UUGGUGUGUUGGAUGAUGGAGU (SEQ ID NO: 15). *let-7c* comprises the sequence UUCGUAUGUUGGAUGAUGGAGU (SEQ ID NO: 16). *let-7d* comprises the sequence UGAUACGUUGGAUGAUGGAGA (SEQ ID NO: 17). *let-7e* comprises the sequence UAUAUGUUGGAGGAUGGAGU (SEQ ID NO: 18). *let-7f* comprises the sequence UUGAUAUGUUAGAUGAUGGAGU (SEQ ID NO: 19). *let-7g* comprises the sequence GACAUGUUUGAUGAUGGAGU (SEQ ID NO: 20). *let-7i* comprises the sequence UGUCGUGUUUGUUGAUGGAGU (SEQ ID NO: 21).

The present invention provides isolated nucleic acids and compositions containing modified oligo molecules including, but not limited to, the preferred modified oligo defined by the sequence UGAGGUAGUAGGUUGUGUGCUUUU (variant nucleic acid residue at position 20 bolded)(SEQ ID NO: 22). For the preceding sequence, thymine (T) may be substituted for uracil (U).

### Isolated Nucleic Acid Molecules

There is herein provided isolated nucleic acid molecules that bind sequences containing presumptive miRNA binding sites, e.g. modified oligonucleotides (referred to herein as "oligos"). These miRNA binding sites contain one or more mutations. These mutations may be SNPs. Exemplary isolated nucleic acid molecules containing one or more SNPs include, but are not limited to, the nucleic acid molecules of SEQ ID NOs: 3, 4, and 13. Isolated nucleic acid molecules containing one or more SNPs disclosed herein may be interchangeably referred to throughout the present text as "SNP-containing nucleic acid molecules". Isolated nucleic acid molecules, modified oligos, may be engineered to bind SNP-containing nucleic acid molecules. Conveniently, isolated nucleic acid molecules, such as modified oligos, bind sequences containing one or more insertions, deletions, inversions, frameshifts, translocations, recombinations, or substitution.

The isolated nucleic acid molecules include single- and double-stranded ribonucleic acid (RNA) and deoxyribonucleic acid (DNA) molecules, as well as all art-recognized analogs, derivatives, or hybrid molecules thereof. Isolated nucleic acid molecules also include reagents for synthesizing modified oligos, such as isolated full-length genes, transcripts, cDNA molecules, primers, vectors, plasmids, endogenous or naturally-occurring miRNAs, and fragments thereof.

As used herein, an "isolated nucleic acid molecule" generally is one that binds a miRNA binding site or miRNA complementary site. Isolated nucleic acid molecules are engineered to bind sequences containing one or more mutations that alter or modify the binding efficacy of at least one endogenous and/or naturally occurring miRNA, and is separated from most other nucleic acids present in the natural source of the nucleic acid molecule. Moreover, an "isolated" nucleic acid molecule can be substantially free of other cellular material, or culture medium when produced by recombinant techniques, or chemical precursors or other chemicals when chemically synthesized. A nucleic acid molecule can be fused to other coding or regulatory sequences and still be considered "isolated". Nucleic acid molecules present in non-human transgenic animals, which do not naturally occur in the animal, are also considered "isolated". For example, recombinant nucleic acid molecules contained in a vector are considered "isolated". Further examples of "isolated" nucleic acid molecules includes recombinant DNA or RNA molecules maintained in heterologous host cells, and purified (partially or substantially) DNA or RNA molecules in solution. Isolated RNA molecules include *in vivo* or *in vitro* RNA transcripts of the isolated nucleic acid molecules of the present invention. Moreover, isolated RNA molecules include, but are not limited to, messenger RNA (mRNA), interfering RNA (RNAi), short interfering RNA (siRNA), short hairpain RNA (siRNA), double-stranded RNA (dsRNA), and microRNA (miRNA). Isolated nucleic acid molecules further include such molecules produced synthetically.

Generally, an isolated nucleic acid molecule comprises one or more sequences engineered to bind to a miRNA binding site containing one or more mutations, with flanking nucleotide sequences on either side of the modified oligo sequence. A flanking sequence can include nucleotide residues that are naturally associated with the targeted miRNA binding site and/or heterologous nucleotide sequences. Preferably the flanking sequence is up to about 500, 300, 100, 60, 50, 30, 25, 20, 15, 10, 8, or 4 nucleotides (or any other length in-between) on either side of the modified oligo sequence, or as long as the full-length gene, entire coding, or non-coding sequence (or any portion thereof, such as, an exon, intron, or a 5' or 3' untranslated region).

Isolated nucleic acids molecules are associated with, bound to, conjugated to, linked to, or incorporated with a virus (or any part or fragment thereof), a liposome, a lipid, an antibody, an intrabody, a protein, a receptor, a ligand, a cytotoxic compound, a radioisotope, a toxin, a chemotherapeutic agent, a salt, an ester, a prodrug, a polymer, a hydrogel, a microcapsule, a nanocapsule, a microsphere, a cyclodextin, a plasmid, an expression vector, a proteinaceous vector, a detectable label (e.g. fluorescent, radioactive, magnetic, paramagnetic, etc.), an antigen, a diluent, an excipient, an adjuvant, an emulsifier, a buffer, a stabilizer, or a preservative.

As used herein, the term "fragment" is meant to describe an isolated nucleic acid molecule that is shorter the isolated nucleic acid molecule from which it is derived. Fragments of isolated nucleic acid molecules can contain, consist of, or comprise any part of the isolated nucleic acid molecule from which it is derived. A fragment typically comprises a contiguous nucleotide sequence at least about 8 or more nucleotides, more preferably at least about 10 or more nucleotides, and even more preferably at least about 16 or more nucleotides. Further, a fragment could comprise at least about 18, 20, 21, 22, 25, 30, 40, 50, 60, 100, 250 or 500 (or any other number in-between) nucleotide in length. The length of the fragment will be based on its intended use. A labeled probe can then be used, for example, to screen a cDNA library, genomic DNA library, or mRNA to isolate nucleic acid corresponding to the region of interest. Further, primers can be used in amplification reactions, such as for purposes of assaying one or more miRNA binding sites or for cloning specific regions of a gene.

An isolated nucleic acid molecule herein described further encompasses a modified or synthetic oligo that is the product of any one of a variety of nucleic acid amplification methods, which are used to increase the copy numbers of a polynucleotide of interest in a nucleic acid sample. Such amplification methods are well known in the art, and they include but are not limited to, polymerase chain reaction (PCR) (U.S. Pat. Nos. 4,683,195; and 4,683,202; PCR Technology: Principles and Applications for DNA Amplification, ed. H. A. Erlich, Freeman Press, NY, N.Y., 1992), ligase chain reaction (LCR) (Wu and Wallace, Genomics 4:560, 1989; Landegren et al., Science 241:1077, 1988), strand displacement amplification (SDA) (U.S. Pat. Nos. 5,270,184; and 5,422,252), transcription-mediated amplification (TMA) (U.S. Pat. No. 5,399,491), linked linear amplification (LLA) (U.S. Pat. No. 6,027,923), and the like, and isothermal amplification methods such as nucleic acid sequence based amplification (NASBA), and self-sustained sequence replication (Guatelli et al., Proc. Natl. Acad. Sci. USA 87: 1874, 1990). Based on such methodologies, a person skilled in the art can readily design primers in any suitable regions 5' and 3' to a SNP disclosed herein.

As used herein, an "amplified polynucleotide" is a isolated nucleic acid molecule whose amount has been increased at least two fold by any nucleic acid amplification method performed *in vitro* as compared to its starting amount in a test sample.

Preferably, an amplified polynucleotide is the result of at least ten fold, fifty fold, one hundred fold, one thousand fold, or even ten thousand fold increase as compared to its starting amount in a test sample. In a typical PCR amplification, a polynucleotide of interest is often amplified at least fifty thousand fold in amount over the unamplified genomic DNA, but the precise amount of amplification needed for an assay depends on the sensitivity of the subsequent detection method used.

Generally, an amplified polynucleotide is at least about 10 nucleotides in length. More typically, an amplified polynucleotide is at least about 16 nucleotides in length. Preferably, an amplified polynucleotide is at least about 2025 nucleotides in length. More preferably, an amplified polynucleotide is at least about 21, 22, 23, 24, 25, 30, 35, 40, 45, 50, or 60 nucleotides in length, or is at least about 100, 200, or 300 nucleotides in length. While the total length of an amplified polynucleotide can be as long as an exon, an intron, a 5' UTR, a 3' UTR, or the entire gene where the altered miRNA binding site of interest resides, an amplified product is typically no greater than about 1,000 nucleotides in length (although certain amplification methods may generate amplified products greater than 1000 nucleotides in length). More preferably, an amplified polynucleotide is not greater than about 600 nucleotides in length.

The amplified product may be at least about 24 nucleotides in length, and binds a SNP-containing let-7 complementary site (LCS). In a specific embodiment of the invention, the amplified product is at least about 24 nucleotides in length, and comprises SEQ ID NO: 22. Such a product may have additional sequences on its 5' end or 3' end or both.

The present disclosure provides isolated nucleic acid molecules that comprise, consist of, or consist essentially of one or more polynucleotide sequences that bind mutated or altered miRNA binding sites and/or fragments thereof.

Accordingly, there is provided nucleic acid molecules that consist of the nucleotide sequence of SEQ ID NOs: 22-36. A nucleic acid molecule consists of a nucleotide sequence when the nucleotide sequence is the complete nucleotide sequence of the nucleic acid molecule.

The present disclosure further provides nucleic acid molecules that consist essentially of the nucleotide sequence of SEQ ID NOs: 22-36. A nucleic acid molecule consists essentially of a nucleotide sequence when such a nucleotide sequence is present with only a few additional nucleotide residues in the final nucleic acid molecule.

The present disclosure further provides nucleic acid molecules that comprise the nucleotide sequence of SEQ ID NOs: 22-36. A nucleic acid molecule comprises a nucleotide sequence when the nucleotide sequence is at least part of the final nucleotide sequence of the nucleic acid molecule. In such a fashion, the nucleic acid molecule can be only the nucleotide sequence or have additional nucleotide residues, such as residues that are naturally associated with it or heterologous nucleotide sequences. Such a nucleic acid molecule can have one to a few additional nucleotides or can comprise many more additional nucleotides. A brief description of how various types of these nucleic acid molecules can be readily made and isolated is provided below, and such techniques are well known to those of ordinary skill in the art (Sambrook and Russell, 2000, Molecular Cloning: A Laboratory Manual, Cold Spring Harbor Press, NY).

The isolated nucleic acid molecules include, but are not limited to, nucleic acid molecules having a sequence encoding a peptide alone, a sequence encoding a mature peptide and additional coding sequences such as a leader or secretory sequence (e.g., a pre-pro or pro-protein sequence), a sequence encoding a mature peptide with or without additional coding sequences, plus additional non-coding sequences, for example introns and non-coding 5' and 3' sequences such as transcribed but untranslated sequences that play a role in, for example, transcription, mRNA processing (including splicing and polyadenylation signals), ribosome binding, and/or stability of mRNA. In addition, the nucleic acid molecules may be fused to heterologous marker sequences encoding, for example, a peptide that facilitates purification.

Isolated nucleic acid molecules can be in the form of RNA, such as mRNA, or in the form DNA, including cDNA and genomic DNA, which may be obtained, for example, by molecular cloning or produced by chemical synthetic techniques or by a combination thereof (Sambrook and Russell, 2000, Molecular Cloning: A Laboratory Manual, Cold Spring Harbor Press, NY). Furthermore, isolated nucleic acid molecules can also be partially or completely in the form of one or more types of nucleic acid analogs, such as peptide nucleic acid (PNA) (U.S. Pat. Nos. 5,539,082; 5,527,675; 5,623,049; 5,714,331). The nucleic

acid, especially DNA, can be double-stranded or single-stranded. Single-stranded nucleic acid can be the coding strand (sense strand) or the complementary non-coding strand (antisense strand). DNA, RNA, or PNA segments can be assembled, for example, from fragments of the human genome (in the case of DNA or RNA) or single nucleotides, short oligonucleotide linkers, or from a series of oligonucleotides, to provide a synthetic nucleic acid molecule. Nucleic acid molecules can be readily synthesized using the sequences provided herein as a preference; oligonucleotide and PNA oligomer synthesis techniques are well known in the art (see, e.g., Corey, "Peptide nucleic acids: expanding the scope of nucleic acid recognition", Trends Biotechnol. 1997 June; 15(6):224-9, and Hyrup et al., "Peptide nucleic acids (PNA): synthesis, properties and potential applications", Bioorg Med Chem. 1996 January; 4(1):5-23). Furthermore, large-scale automated oligonucleotide/PNA synthesis (including synthesis on an array or bead surface or other solid support) can readily be accomplished using commercially available nucleic acid synthesizers, such as the Applied Biosystems (Foster City, Calif.) 3900 High-Throughput DNA Synthesizer or Expedite 8909 Nucleic Acid Synthesis System, and the sequence information provided herein.

There is herein described nucleic acid analogs that contain modified, synthetic, or non-naturally occurring nucleotides or structural elements or other alternative/modified nucleic acid chemistries known in the art. Such nucleic acid analogs are useful, for example, as detection reagents (e.g., primers/probes). Furthermore, kits/systems (such as beads, arrays, etc.) that include these analogs are also described herein. For example, PNA oligomers that are based on the polymorphic sequences of the present disclosure are specifically contemplated. PNA oligomers are analogs of DNA in which the phosphate backbone is replaced with a peptide-like backbone (Lagriffoul et al., Bioorganic & Medicinal Chemistry Letters, 4: 1081-1082 (1994), Petersen et al., Bioorganic & Medicinal Chemistry Letters, 6: 793-796 (1996), Kumar et al., Organic Letters 3(9): 1269-1272 (2001), W096/04000). PNA hybridizes to complementary RNA or DNA with higher affinity and specificity than conventional oligonucleotides and oligonucleotide analogs. The properties of PNA enable novel molecular biology and biochemistry applications unachievable with traditional oligonucleotides and peptides.

The term "modified or synthetic oligonucleotide (oligo) molecule" is not limited to molecules containing only naturally-occurring RNA or DNA, but also encompasses chemically-modified nucleotides and non-nucleotides.

The modified oligo molecules may lack 2'-hydroxy (2'-OH) containing nucleotides. Modified oligos do not require the presence of nucleotides having a 2'-hydroxy group for mediating gene silencing and as such, isolated nucleic acid molecules, e.g. modified oligos, optionally do not include any ribonucleotides (e.g., nucleotides having a 2'-OH group). Such oligo molecules that do not require the presence of ribonucleotides within the oligo molecule to support gene silencing can however have an attached linker or linkers or other attached or associated groups, moieties, or chains containing one or more nucleotides with 2'0H groups. Optionally, miRNA molecules can comprise ribonucleotides at about 5, 10, 20, 3 0, 40, or 50% of the nucleotide positions.

As used herein, the term "modified oligo" is meant to be equivalent to other terms used to describe nucleic acid molecules that are capable of mediating sequence specific gene silencing or interference, e.g., microRNA (miRNA), short interfering RNA (siRNA), double-stranded RNA (dsRNA), interfering RNA (RNAi), short hairpin RNA (shRNA), short interfering oligonucleotide, short interfering nucleic acid, short interfering modified oligonucleotide, chemically-modified siRNA, post-transcriptional gene silencing RNA (ptgsRNA), and other art-recognized equivalents. As used herein, the term "gene silencing" is meant to describe the downregulation, knock-down, degradation, inhibition, suppression, repression, prevention, or decreased expression of a gene, transcript and/or polypeptide product. Gene silencing and interference also describe the prevention of translation of mRNA transcripts into a polypeptide. Translation is prevented, inhibited, or decreased by degrading mRNA transcripts or blocking mRNA translation.

Modified oligo molecules, or precursors thereof, may comprise separate sense and antisense sequences or regions, wherein the sense and antisense regions are covalently linked by nucleotide or non-nucleotide linker molecules, or are alternately non-covalently linked by ionic interactions, hydrogen bonding, van der waals interactions, hydrophobic interactions, and/or stacking interactions.

As used herein the term "antisense RNA" is an RNA strand having a sequence complementary to a target gene mRNA, and thought to induce gene silencing or interference by binding to the target gene mRNA. As used herein the term "Sense RNA" has a sequence complementary to the antisense RNA, and when annealed to its complementary antisense RNA, forms a siRNA. Antisense and sense RNAs are conventionally synthesized with an RNA synthesizer.

Modified oligos are assembled from two separate oligonucleotides, where one strand is the sense strand and the other is the antisense strand, wherein the antisense and sense strands are self-complementary (i.e., each strand comprises nucleotide sequence that is complementary to nucleotide sequence in the other strand; such as where the antisense strand and sense strand form a duplex or double stranded structure, e.g., wherein the double stranded region is about 1, 2, 5, 10, 15, or 19 base pairs, or any value in between). The antisense strand may comprise a nucleotide sequence that is complementary to a nucleotide sequence in a target nucleic acid molecule or a portion thereof, and the sense strand may comprise a nucleotide sequence corresponding to the target nucleic acid sequence or a portion thereof. Alternatively, the modified oligo is assembled from a single oligonucleotide, where the self-complementary sense and antisense regions of the oligo are linked by means of a nucleic acid-based or non-nucleic acid-based linker(s). The modified oligo is assembled as a single oligonucleotide representing the antisense strand.

Modified oligos engineered for intracellular delivery according to the methods and compositions described herein include a polynucleotide with a duplex, asymmetric duplex, hairpin or asymmetric hairpin secondary structure, having self-complementary sense and antisense regions, wherein the antisense region comprises a nucleotide sequence that is complementary to a nucleotide sequence in a separate target nucleic acid molecule or a portion thereof, and the sense region comprises a nucleotide sequence corresponding to the target nucleic acid sequence or a portion thereof.

Non-limiting examples of chemical modifications that are made in a modified oligo include without limitation phosphorothioate internucleotide linkages, 2'- deoxyribonucleotides, 2'-0-methyl ribonucleotides, 2'-deoxy-2'-fluoro ribonucleotides, "universal base" nucleotides, "acyclic" nucleotides, 5-C-methyl nucleotides, and terminal glyceryl and/or inverted deoxy abasic residue incorporation. These chemical modifications, when used in modified oligos, preserve silencing or interference activity in cells while at the same time, dramatically increasing the serum stability of these compounds.

In a non-limiting example, the introduction of chemically-modified nucleotides into nucleic acid molecules provides a powerful tool in overcoming potential limitations of in vivo stability and bioavailability inherent to native RNA molecules that are delivered exogenously. For example, the use of chemically-modified nucleic acid molecules can enable a lower dose of a particular nucleic acid molecule for a given therapeutic effect sine chemically-modified nucleic acid molecules tend to have a longer half-life in serum. Furthermore, certain chemical modifications can improve the bioavailability of nucleic acid molecules by targeting particular cells or tissues and/or improving cellular uptake of the nucleic acid molecule. Therefore, even if the activity of a chemically-modified nucleic acid molecule is reduced as compared to a native nucleic acid molecule, e.g., when compared to an all-RNA nucleic acid molecule, the overall activity of the modified nucleic acid molecule can be greater than that of the native molecule due to improved stability and/or delivery of the molecule. Unlike native unmodified oligos, chemically-modified oligos can also minimize the possibility of activating interferon activity in humans.

The antisense region of a modified oligo molecule of the disclosure can comprise a phosphorothioate internucleotide linkage at the 3'-end of said antisense region. Optionally, the antisense region can comprise about one to about five phosphorothioate internucleotide linkages at the 5'-end of said antisense region. Optionally, the 3'-terminal nucleotide overhangs of a modified oligo molecule can comprise ribonucleotides or deoxyribonucleotides that are chemically-modified at a nucleic acid sugar, base, or backbone. Optionally, the 3'-terminal nucleotide overhangs can comprise one or more universal base ribonucleotides. Optionally, the 3'-terminal nucleotide overhangs can comprise one or more acyclic nucleotides.

For example, in a non-limiting example, a chemically-modified modified oligo may have about 1, 2, 3, 4, 5, 6, 7, 8 or more phosphorothioate internucleotide linkages in one variant miRNA strand. A chemically-modified modified oligo individually may have about 1, 2, 3, 4, 5, 6, 7, 8 or more phosphorothioate internucleotide linkages in both variant miRNA strands. The phosphorothioate internucleotide linkages can be present in one or both oligonucleotide strands of the modified oligo duplex, e.g., in the sense strand, the antisense strand, or both strands. The modified oligo molecules can comprise one or more phosphorothioate internucleotide linkages at the 3'-end, the 5'-end, or both of the 3'- and 5'-ends of the sense strand, the antisense strand, or both strands. An exemplary modified oligo molecule can comprise about 1 to about 5 or more (e.g., about 1, 2, 3, 4, 5, or more) consecutive phosphorothioate internucleotide linkages at the 5'-end of the sense strand, the antisense strand, or both strands. In another non-limiting example, modified oligo molecules can comprise one or more (e.g., about 1, 2, 3, 4, 5, 6, 7, 8, 9, 10, or more) pyrimidine phosphorothioate internucleotide linkages in the sense strand, the antisense strand, or both strands. In yet another non-limiting example, modified oligo molecules of the invention can comprise one or more (e.g., about 1, 2, 3, 4, 5, 6, 7, 8, 9, 10, or more) purine phosphorothioate internucleotide linkages in the sense strand, the antisense strand, or both strands.

A modified oligo molecule may be comprised of a circular nucleic acid molecule, wherein the modified oligo is about 38 to about 70 (e.g., about 38, 40, 45, 50, 55, 60, 65, or 70) nucleotides in length having about 18 to about 23 (e.g., about 18, 19, 20, 21, 22, or 23) base pairs wherein the circular oligonucleotide forms a dumbbell shaped structure having about 19 base pairs and 2 loops.

A circular modified oligo molecule contains two loop motifs, wherein one or both loop portions of the modified oligo molecule is biodegradable. For example, a circular modified oligo molecule of this disclosure is designed such that degradation of the loop portions of the modified oligo molecule in vivo can generate a double-stranded modified oligo molecule with 3'-terminal overhangs, such as 3'-terminal nucleotide overhangs comprising about 2 nucleotides.

Modified nucleotides present in modified oligo molecules, preferably in the antisense strand of the modified oligos, but also optionally in the sense and/or both antisense and sense strands, comprise modified nucleotides having properties or characteristics similar to naturally occurring ribonucleotides. For example, the disclosure provides modified oligo molecules including modified nucleotides having a northern conformation (e.g., northern pseudorotation cycle, see, e.g., Saenger, Principles of Nucleic Acid Structure, Springer-Verlag Ed., 1984). As such, chemically modified nucleotides present in the modified oligos, preferably in the antisense strand of the modified oligo molecules, but also optionally in the sense and/or both antisense and sense strands, are resistant to nuclease degradation while at the same time maintaining the capacity to mediate silencing or interference. Non-limiting examples of nucleotides having a northern configuration include locked nucleic acid (LNA) nucleotides (e.g., 2'-O, 4'-C-methylene-(D-ribofuranosyl) nucleotides); 2'- methoxyethoxy (MOE) nucleotides; 2'-methyl-thio-ethyl, 2'-deoxy-2'-fluoro nucleotides. 2'-deoxy-2'-chloro nucleotides, 2'-azido nucleotides, and 2'-0-methyl nucleotides.

The sense strand of a double stranded modified oligo molecule may have a terminal cap moiety such as an inverted deoxybasic moiety, at the 3'-end, 5'-end, or both 3' and 5'-ends of the sense strand.

A modified oligo is further comprised of a nucleotide, non-nucleotide, or mixed nucleotide/non-nucleotide linker that joins the sense region of the modified oligo to the antisense region of the modified oligo. A nucleotide linker can be a linker of >2 nucleotides in length, e.g., about 3, 4, 5, 6, 7, 8, 9, or 10 nucleotides in length. The nucleotide linker can be a nucleic acid aptamer. By "aptamer" or "nucleic acid aptamer" as used herein is meant a nucleic acid molecule that binds specifically to a target molecule wherein the nucleic acid molecule has sequence that comprises a sequence recognized by the target molecule in its natural setting. Alternately, an aptamer can be a nucleic acid molecule that binds to a target molecule where the target molecule does not naturally bind to a nucleic acid. The target molecule can be any molecule of interest For example, the aptamer can be used to bind to a ligand binding domain of a protein, thereby preventing interaction of the naturally occurring ligand with the protein. This is a non-limiting example and those in the art will recognize that other embodiments can be readily generated using techniques generally known in the art. (See, e.g., Gold, et al, Annu. Rev. Biochem. 64:763, 1995; Brody and Gold, J. Biotechnol 74:5, 2000; Sun, Curr. Opin. Mol. Ther. 2:100, 2000; Kusser, J. Biotechnol. 74:27, 2000; Hermann and Patel, Science 287:820, 2000; and Jayasena, Clinical Chemistry 45:1628, 1999.

A non-nucleotide linker may be comprised of an abasic nucleotide, polyether, polyamine, polyamide, peptide, carbohydrate, lipid, polyhydrocarbon, or other polymeric compounds (e.g., polyethylene glycols such as those having from 2 to 100 ethylene glycol units). Specific examples include those described by Seela and Kaiser, Nucleic Acids Res. 18:6353, 1990 and Nucleic Acids Res. 15:3113, 1987; Cload and Schepartz, J. Am. Chem. Soc. 113:6324, 1991; Richardson and Schepartz, J. Am. Chem. Soc. 113:5109, 1991; Ma, et al., Nucleic Acids Res. 21:2585, 1993 and Biochemistry 32:1751, 1993; Durand, et al., Nucleic Acids Res. 18:6353, 1990; McCurdy, et al., Nucleosides & Nucleotides 10:287, 1991; Jschke, et al., Tetrahedron Lett. 34:301, 1993; Ono, et al., Biochemistry 30:9914 (1991); Arnold, et al., International Publication No. WO 89/02439; Usman, et al., International Publication No. WO 95/06731; Dudycz, et al., International Publication No. WO 95/11910 and Ferentz and Verdine, J. Am. Chem. Soc. 113:4000, 1991. A "non-nucleotide" further means any group or compound that can be incorporated into a nucleic acid chain in the place of one or more nucleotide units, including either sugar and/or phosphate substitutions, and allows the remaining bases to exhibit their enzymatic activity. The group or compound can be abasic in that it does not contain a commonly recognized nucleotide base, such as adenosine, guanine, cytosine, uracil or thymidine, e.g., at the Cl position of the sugar.

Additional examples of nucleic acid modifications that improve the binding properties and/or stability of a nucleic acid include the use of base analogs such as inosine, intercalators (U.S. Pat. No. 4,835,263) and the minor groove binders (U.S. Pat. No. 5,801,115). Thus, references herein to nucleic acid molecules include PNA oligomers and other nucleic acid analogs. Other examples of nucleic acid analogs and alternative/modified nucleic acid chemistries known in the art are described in Current Protocols in Nucleic Acid Chemistry, John Wiley & Sons, N.Y. (2002). Isolated nucleic acids of the disclosure are comprised of base analogs including, but not limited to, any of the known base analogs of DNA and RNA such as, but not limited to 4-acetylcytosine, 8- hydroxy-N-6-methyladenosine, aziridinylcytosine, pseudoisocytosine, 5- (carboxyhydroxylmethyl) uracil, 5-bromouracil, 5-carboxymethylaminomethyl-2- thiouracil, 5-carboxymethylaminomethyluracil, dihydrouracil, inosine, N6- isopentenyladenine, 1-methyladenine, 1-methylpseudouracil, 1-methyl guanine, 1- methylinosine, 2,2-dimethylguanine, 2-methyladenine, 2-methylguanine, 3- methylcytosine, 5-methylcytosine, N6-methyladenine, 7-methylguanine, 5- methylaminomethyluracil, 5-methoxy-aminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2- thiouracil, 4-thiouracil, 5-methyluracil, N-uracil-5-oxyacetic acid methylester, 2,6-diaminopurine, and 2'-modified analogs such as, but not limited to 0-methyl, amino-, and fluoro-modified analogs.

The modified or synthetic oligos and compositions of the disclosure are modified to enhance stability by modification with nuclease resistant groups, e.g., 2'-amino, 2'-Callyl, 2'-fluoro, 2'-0-methyl, and 2'-H. (For a review see Usman and Cedergren, TIBS 17:34, 1992; Usman, et al., Nucleic Acids Symp. Ser. 31:163, 1994). Modified oligos and compositions are purified by gel electrophoresis using general methods or can be purified by high pressure liquid chromatography and re-suspended in water.

Chemically synthesizing nucleic acid molecules with modifications (base, sugar and/or phosphate) prevents their degradation by serum ribonucleases, which increases their potency. See, e.g., Eckstein, et al., International Publication No. WO 92/07065; Perrault, et al., Nature 344:565, 1990; Pieken, et al., Science 253:314, 1991; Usman and Cedergren, Trends in Biochem. Sci. 17:334, 1992; Usman, et al, International Publication No. WO 93/15187; and Rossi, et al., International Publication No. WO 91/03162; Sproat, U.S. Pat. No. 5,334,711; Gold, et al., U.S. Pat. No. 6,300,074. All of the above references describe various chemical modifications that are made to the base, phosphate and/or sugar moieties of the isolated nucleic acid molecules described herein.

There are several examples in the art describing sugar, base and phosphate modifications that are introduced into isolated nucleic acid molecules of the disclosure with significant enhancement in their nuclease stability and efficacy. For example, oligonucleotides are modified to enhance stability and/or enhance biological activity by modification with nuclease resistant groups, e.g., T-amino, 2'-C-allyl, 2'-fluoro, 2'-0- methyl, 2'-H, nucleotide base modifications. For a review see Usman and Cedergren, TIBS 17:34, 1992; Usman, et al., Nucleic Acids Symp. Ser. 31:163, 1994; Burgin, et al., Biochemistry 35:14090, 1996. Sugar modification of nucleic acid molecules have been extensively described in the art. See Eckstein, et al., International Publication PCT No. WO 92/07065; Perrault, et al., Nature 344:565-568, 1990; Pieken, et al., Science 253:314-317, 1991; Usman and Cedergren, Trends in Biochem. Sci. 17:334339, 1992; Usman, et al., International Publication PCT No. WO 93/15187; Sproat, U.S. Pat. No. 5,334,711 and Beigelman, et al., J. Biol. Chem. 270:25702, 1995; Beigelman, et al., International PCT publication No. WO 97/26270; Beigelman, et al., U.S. Pat. No. 5,716,824; Usman, et al., U.S. Pat. No. 5,627,053; Woolf, et al., International PCT Publication No. WO 98/13526; Thompson, et al., Karpeisky, et al., Tetrahedron Lett. 39:11131, 1998; Earnshaw and Gait, Biopolymers (Nucleic Acid Sciences) 48:39-55, 1998; Verma and Eckstein, Annu. Rev. Biochem. 67:99-134,1998; and Burlina, et al, Bioorg. Med. Chem. 5:1999-2010, 1997. Such publications describe general methods and strategies to determine the location of incorporation of sugar, base and/or phosphate modifications and the like into nucleic acid molecules without modulating catalysis. In view of such teachings, similar modifications are uses as described herein to modify the oligonucleotide molecules of the disclosure so long as the ability of the modified oligos to promote gene silencing in cells is not significantly inhibited.

While chemical modification of oligonucleotide internucleotide linkages with phospharathioate, phosphorodithioate, and/or 5'-methylphosphonate linkages improves stability, excessive modifications can cause some toxicity or decreased activity. Therefore, when engineering isolated nucleic acid molecules of the disclosure, the amount of these internucleotide linkages are minimized. The reduction in the concentration of these linkages lowers toxicity, resulting in increased efficacy and higher specificity of these molecules.

There is provided modified or synthetic oligo molecules, with phosphate backbone modifications comprising one or more phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, and/or alkylsilyl, substitutions. For a review of oligonucleotide backbone modifications, see Hunziker and Leumann, "Nucleic Acid Analogues: Synthesis and Properties, in Modem Synthetic Methods," VCH, 331-417, 1995, and Mesmaeker, et al, "Novel Backbone Replacements for Oligonucleotides, in Carbohydrate Modifications in Antisense Research," ACS, 24-39, 1994.

Further variants of the nucleic acid molecules including, but not limited to those identified as SEQ ID NOs: 14-36, such as naturally occurring allelic variants (as well as orthologs and paralogs) and synthetic variants produced by mutagenesis techniques, can be identified and/or produced using methods well known in the art. Such further variants can comprise a nucleotide sequence that shares at least 70-80%, 80-85%, 85-90%, 91%, 92%, 93%, 94%, 95%, 96%, 97%, 98%, or 99% sequence identity with a nucleic acid sequence disclosed as SEQ ID NOs: 14-36 (or a fragment thereof). Thus, the present disclosure specifically contemplates isolated nucleic acid molecule that have a certain degree of sequence variation compared with the sequences of SEQ ID NOs: 14-36.

The modified oligos and compositions of the disclosure are routinely made through techniques such as solid phase synthesis. Equipment for such synthesis is sold by several vendors including, for example, Applied Biosystems, (Foster City, Calif.). Any other means for such synthesis known in the art is additionally or alternatively employed. It is well known to use similar techniques to prepare oligonucleotides such as the phosphorothioates and alkylated derivatives.

Oligonucleotides (e.g., certain modified oligonucleotides or portions of oligonucleotides lacking ribonucleotides) are synthesized using protocols known in the art, e.g., as described in Caruthers, et al., Methods in Enzymology 211:3-19, 1992; Thompson, et al., International PCT Publication No. WO 99/54459; Wincott, et al., Nucleic Acids Res. 23:2677-2684, 1995; Wincott, et al., Methods Mol. Bio. 74:59, 1997; Brennan, et al., Biotechnol Bioeng. 61:33-45, 1998; and Brennan, U.S. Pat. No. 6,001,311. Synthesis of RNA, including certain modified oligo molecules of the disclosure, follows general procedures as described, e.g., in Usman, et al, J. Am. Chem Soc. 109:7845, 1987; Scaringe, et al., Nucleic Acids Res. 18:5433, 1990; and Wincott, et al., Nucleic Acids Res. 23:2677-2684, 1995; Wincott, et al., Methods Mol. Bio. 74:59, 1997.

The comparison of sequences and determination of percent identity between two sequences can be accomplished using a mathematical algorithm. (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Preferably, the percent identity between two amino acid sequences is determined using the Needleman and Wunsch algorithm (J. Mol. Biol. (48):444-453 (1970)) which has been incorporated into the GAP program in the GCG software package, using either a Blossom 62 matrix or a PAM250 matrix, and a gap weight of 16, 14, 12, 10, 8, 6, or 4 and a length weight of 1, 2, 3, 4, 5, or 6.

Preferably, the percent identity between two nucleotide sequences is determined using the GAP program in the GCG software package (Devereux, J., et al., Nucleic Acids Res. 12(1):387 (1984)), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Myers and W. Miller (CABIOS, 4:11-17 (1989)) which has been incorporated into the ALIGN program (version 2.0), using a PAM120 weight residue table, a gap length penalty of 12, and a gap penalty of 4.

The nucleotide and amino acid sequences disclosed herein can further be used as a "query sequence" to perform a search against sequence databases to, for example, identify other family members or related sequences. Such searches can be performed using the NBLAST and )(BLAST programs (version 2.0) of Altschul, et al. (J. Mol. Biol. 215:403-10 (1990)). BLAST nucleotide searches can be performed with the NBLAST program, score=100, wordlength=12 to obtain nucleotide sequences homologous to the nucleic acid molecules described herein: BLAST protein searches can be performed with the XBLAST program, score=50, wordlength=3 to obtain amino acid sequences, homologous to the proteins of the disclosure. To obtain gapped alignments for comparison purposes, Gapped BLAST can be utilized as described in Altschul et al. (Nucleic Acids Res. 25(17):3389-3402 (1997)). When utilizing BLAST and gapped BLAST programs, the default parameters of the respective programs (e.g., )(BLAST and NBLAST) can be used. In addition to BLAST, examples of other search and sequence comparison programs used in the art include, but are not limited to, FASTA (Pearson, Methods Mol. Biol. 25, 365-389 (1994)) and KERR (Dufresne et al., Nat Biotechnol 2002 December; 20(12): 1269-71). For further information regarding bioinformatics techniques, see Current Protocols in Bioinfonnatics, John Wiley & Sons, Inc., N.Y.

### Therapeutic Methods

The nucleic acid molecules described herein are used to modify the expression of genes containing mutations that lead to altered wild type miRNA binding, and thus, the development of a variety of disorders. Exemplary disorders include but are not limited to, developmental, aging, inflammatory, degenerative, metabolic, proliferative, circulatory; cognitive, productive, and behavioral disorders. Preferably, the disorder is cancer. For example, the isolated nucleic acid molecules bind altered or mutated miRNA binding sites with a binding efficacy that equals or exceeds the binding efficacy achieved by at least one endogenous or naturally-occurring miRNA when bound to the corresponding wild typeor altered/mutated mRNA binding site. Isolated nucleic acid molecules used to modify gene expression may be modified oligos.

A therapeutically effective amount of a composition disclosed herein is an amount of a modified oligo, or a precursor thereof, that when administered to a subject, results in the silencing, or decreased expression, of at least one gene or mRNA transcript. The effectiveness of administration of a Pharmaceutical composition may be measured by testing a subject, e.g. biopsied tissue or a bodily fluid, for decreased gene expression using art-recognized methods.

Alternatively, or in addition, a pharmaceutically effective amount of a composition is an amount of a modified oligo, or a precursor thereof, that prevents, inhibits the occurrence or reoccurrence of, treats, or alleviates a sign or symptom (to some extent) of a disorder. Preferably, a pharmaceutically effective dose is that dose required to alleviate at least one sign or symptom of cancer. As used herein, the term "treat" is meant to describe a process by which a sign or symptom of a disorder, such as cancer, is eliminated. Alternatively, or in addition, a disorder such as cancer, which can occur in multiple locations, is treated if the cancer is eliminated within at least one of multiple locations.

As used herein, the term "alleviate" is meant to describe a process by which the severity of a sign or symptom of a disorder is decreased. Importantly, a sign or symptom can be alleviated without being eliminated. Preferably, the administration of pharmaceutical compositions described herein leads to the elimination of a sign or symptom, however, elimination is not required. Effective dosages are expected to decrease the severity of a sign or symptom. For instance, a sign or symptom of a disorder such as cancer, which can occur in multiple locations, is alleviated if the severity of the cancer is decreased within at least one of multiple locations.

As used herein, the term "severity" is meant to describe the potential of cancer to transform from a precancerous, or benign, state into a malignant state. Alternatively, or in addition, severity is meant to describe a cancer stage, for example, according to the TNM system (accepted by the International Union Against Cancer (UICC) and the American Joint Committee on Cancer (AJCC)) or by other art-recognized methods. Cancer stage refers to the extent or severity of the cancer, based on factors such as the location of the primary tumor, tumor size, number of tumors, and lymph node involvement (spread of cancer into lymph nodes). Alternatively, or in addition, severity is meant to describe the tumor grade by art-recognized methods (see, National Cancer Institute, www.cancer.gov). Tumor grade is a system used to classify cancer cells in terms of how abnormal they look under a microscope and how quickly the tumor is likely to grow and spread. Many factors are considered when determining tumor grade, including the structure and growth pattern of the cells. The specific factors used to determined tumor grade vary with each type of cancer. Severity also describes a histologic grade, also called differentiation, which refers to how much the tumor cells resemble normal cells of the same tissue type (see, National Cancer Institute, www.cancer.gov). Furthermore, severity describes a nuclear grade, which refers to the size and shape of the nucleus in tumor cells and the percentage of tumor cells that are dividing (see, National Cancer Institute, www.cancer.gov).

In another aspect of the disclosure, severity describes the degree to which a tumor has secreted growth factors, degraded the extracellular matrix, become vascularized, lost adhesion to juxtaposed tissues, or metastasized. Moreover, severity describes the number of locations to which a primary tumor has metastasized. Finally, severity includes the difficulty of treating tumors of varying types and location. For example, inoperable tumors, those cancers which have greater access to multiple body systems (hematological and immunological tumors), and those which are the most resistant to traditional treatments are considered most severe. In these situations, prolonging the life expectancy of the subject and/or reducing pain, decreasing the proportion of cancerous cells or restricting cells to one system, and improving cancer stage/tumor grade/histological grade/nuclear grade are considered alleviating a sign or symptom of the cancer.

In one aspect of the disclosure, a therapeutically effective amount of a composition described herein is an amount of a modified oligo, or a precursor thereof, that provides a preventative benefit to the subject. As used herein, the term "preventative benefit" is meant to describe a delay in the development or decrease of the severity of a sign or symptom of a disorder, such as cancer.

The pharmaceutically effective dose depends on the type of disease, the composition used, the route of administration, the individual and physical characteristics of the subject under consideration (for example, age, gander, weight, diet, smoking-habit, exercise-routine, genetic background, medical history, hydration, blood chemistry), concurrent medication, and other factors that those skilled in the medical arts will recognize.

Generally, an amount from about 0.01 mg/kg and 25 mg/kg body weight/day of active ingredients is administered dependent upon potency of the negatively charged polymer, e.g. the modified oligo composition. Alternatively, dosage ranges include, but are not limited to, 0.01-0.1 mg/kg, 0.01-1 mg/kg, 0.01-10 mg/kg, 0.01-20 mg/kg, 0.01-30 mg/kg, 0.01-40 mg/kg, 0.01-50 mg/kg, 0.01-60 mg/kg, 0.01-70 mg/kg, 0.01-80 mg/kg; 0.01-90 mg/kg, 0.01-100 mg/kg, 0.01-150 mg/kg, 0.01-200 mg/kg, 0.01-250 mg/kg, 0.01-300 mg/kg, 0.01-500 mg/kg, and all ranges and points in between. Alternatively, dosage ranges include, but are not limited to, 0.01-1 mg/kg, 1-10 mg/kg, 10-20 mg/kg, 20-30 mg/kg, 30-40 mg/kg, 40-50 mg/kg, 50-60 mg/kg, 60-70 mg/kg, 70-80 mg/kg, 80-90 mg/kg, 90-100 mg/kg, 100-150 mg/kg, 150-200 mg/kg, 200-300 mg/kg, 300-500 mg/kg, and all ranges and points in between.

The blood plasma concentration of a composition or a modified oligonucleotide can be about 0.1 µM to about 1000 µM, about 0.1 µM to about 1 µM; about 0.1 µM to about 10 µM; about 10 µM to about 100 µM; about 100 µM to about 500 µM, about 500 µM to about 1000 µM, and any micromolar concentration in between. Alternatively, or in addition, the cerebral spinal fluid concentration of a composition or a modified oligonucleotide can be about 0.1 µM to about 1000 µM, about 0.1 µM to about 1 µM; about 0.1 µM to about 10 µM; about 10 µM to about 100 µM; about 100 µM to about 500 µM, about 500 µM to about 1000 µM, or any micromolar concentration in between.

The pharmaceutical composition can be administered at a dosage from about 1 mg/m² to 5000 mg/m² per day, about 1 mg/m² to 10 mg/m² per day, about 10 mg/m² to 100 mg/m² per day, about 100 to 1000 mg/m² per day, about 1000 to 2500 mg/m² per day, about 2500 to 5000 mg/m² per day, or any daily mg/m² dosage in between Preferably, 1 mg/m² to 5000 mg/m² per day is the administered dosage for a human.

The disclosure provides methods of treating or alleviating a symptom of a cell proliferative disorder. Exemplary cell proliferative disorders encompass a variety of conditions wherein cell division is deregulated. Exemplary cell proliferative disorder include, but are not limited to, neoplasms, benign tumors, malignant tumors, pre-cancerous conditions, *in situ* tumors, encapsulated tumors, metastatic tumors, liquid tumors, solid tumors, immunological tumors, hematological tumors, cancers, carcinomas, leukemias, lymphomas, sarcomas, and rapidly dividing cells. The term "rapidly dividing cell" as used herein is defined as any cell that divides at a rate that exceeds or is greater than what is expected or observed among neighboring or juxtaposed cells within the same tissue.

Preferably, the methods provided herein are used to treat or alleviate a symptom of cancer. Exemplary cancers include, but are not limited to, acute lymphoblastic leukemia, acute myeloid leukemia, adrenocortical carcinoma, adrenocortical carcinoma, AIDS-related cancers, AIDS-related lymphoma, anal cancer, appendix cancer, childhood cerebellar astrocytoma, childhood cerebral astrocytoma, basal cell carcinoma, skin cancer (nonmelanoma), extrahepatic bile duct cancer, bladder cancer, bone cancer, osteosarcoma and malignant fibrous histiocytoma, brain tumor, brain stem glioma, cerebellar astrocytoma, cerebral astrocytoma/malignant glioma, ependymoma, medulloblastoma, supratentorial primitive neuroectodeimal tumors, visual pathway and hypothalamic glioma, breast cancer; bronchial adenomas/carcinoids, carcinoid tumor, gastrointestinal, central nervous system lymphoma, cervical cancer, childhood cancers, chronic lymphocytic leukemia, chronic myelogenous leukemia, chronic myeloproliferative disorders, colon cancer, colorectal cancer, cutaneous T-cell lymphoma, mycosis fungoides, Seziary Syndrome, endometrial cancer, esophageal cancer, extracranial germ cell tumor, extragonadal germ cell tumor, extrahepatic bile duct cancer, eye cancer, intraocular melanoma, retinoblastoma, gallbladder cancer, gastric (stomach) cancer, gastrointestinal carcinoid tumor, gastrointestinal stromal tumor (GIST), germ cell tumor, ovarian germ cell tumor, gestational trophoblastic tumor glioma, head and neck cancer, hepatocellular (liver) cancel, Hodgkin lymphoma, hypopharyngeal cancer, intraocular melanoma, islet cell tumors (endocrine pancreas), Kaposi Sarcoma, kidney (renal cell) cancer, kidney cancer, laryngeal cancer, acute lymphoblastic leukemia, acute myeloid leukemia, chronic lymphocytic leukemia, chronic myelogenous leukemia, hairy cell leukemia, lip and oral cavity cancer, liver cancer, non-small cell lung cancer, small cell lung cancer, AIDS-related lymphoma, non-Hodgkin lymphoma, primary central nervous system lymphoma, Waldenstram macroglobulinemia, medulloblastoma, melanoma, intraocular (eye) melanoma, merkel cell carcinoma, mesothelioma malignant, mesothelioma, metastatic squamous neck cancer, mouth cancer, multiple endocrine neoplasia syndrome, mycosis fungoides, myelodysplastic syndromes, myelodysplastic/ myeloproliferative diseases, chronic myclogenous leukemia, acute myeloid leukemia, multiple myeloma, chronic myeloproliferative disorders, nasopharyngeal cancer, neuroblastoma, oral cancer, oral cavity cancer, oropharyngeal cancer, ovarian cancer, ovarian epithelial cancer, ovarian low malignant potential tumor, pancreatic cancer, islet cell pancreatic cancer, paranasal sinus and nasal cavity cancer, parathyroid cancer, penile cancer, pharyngeal cancer, pheochromocytoma, pineoblastoma and supratentorial primitive neuroectodermal tumors, pituitary tumor, plasma cell neoplasm/multiple myeloma, pleuropulmonary blastoma, prostate cancer, rectal cancer, renal pelvis and ureter, transitional cell cancer, retinoblastoma, rhabdomyosarcoma, salivary gland cancer, ewing family of sarcoma tumors, Kaposi Sarcoma, soft tissue sarcoma, uterine sarcoma, skin cancer (nonmelanoma), skin cancer (melanoma), merkel cell skin carcinoma, small intestine cancer, soft tissue sarcoma, squamous cell carcinoma, stomach (gastric) cancer, supratentorial primitive neuroectodermal tumors, testicular cancer, throat cancer, thymoma, thymoma and thymic carcinoma, thyroid cancer, transitional cell cancer of the renal pelvis and ureter, gestational trophoblastic tumor, urethral cancer, endometrial uterine cancer, uterine sarcoma, vaginal cancer, vulvar cancer, and Wilms Tumor.

As used herein the term "symptom" is defined as an indication of disease, illness, injury, or that something is not right in the body. Symptoms are felt or noticed by the individual experiencing the symptom, but may not easily be noticed by others. Others are defined as non-health-care professionals.

As used herein the term "sign" is also defined as an indication that something is not right in the body. But signs are defined as things that can be seen by a doctor, nurse, or other health care professional.

Cancer is a group of diseases that may cause almost any sign or symptom. The signs and symptoms will depend on where the cancer is, the size of the cancer, and how much it affects the nearby organs or structures. If a cancer spreads (metastasizes), then symptoms may appear in different parts of the body.

As a cancer grows, it begins to push on nearby organs, blood vessels, and nerves. This pressure creates some of the signs and symptoms of cancer. If the cancer is in a critical area, such as certain parts of the brain, even the smallest tumor can cause early symptoms.

But sometimes cancers start in places where it does not cause any symptoms until the cancer has grown quite large. Pancreas cancers, for example, do not usually grow large enough to be felt from the outside of the body. Some pancreatic cancers do not cause symptoms until they begin to grow around nearby nerves (this causes a backache). Others grow around the bile duct, which blocks the flow of bile and leads to a yellowing of the skin known as jaundice. By the time a pancreatic cancer causes these signs or symptoms, it has usually reached an advanced stage.

A cancer may also cause symptoms such as fever, fatigue, or weight loss. This may be because cancer cells use up much of the body's energy supply or release substances that change the body's metabolism. Or the cancer may cause the immune system to react in ways that produce these symptoms.

Sometimes, cancer cells release substances into the bloodstream that cause symptoms not usually thought to result from cancers. For example, some cancers of the pancreas can release substances which cause blood clots to develop in veins of the legs. Some lung cancers make hormone-like substances that affect blood calcium levels, affecting nerves and muscles and causing weakness and dizziness.

Cancer presents several general signs or symptoms that occur when a variety of subtypes of cancer cells are present. Most people with cancer will lose weight at some time with their disease. An unexplained (unintentional) weight loss of 10 pounds or more may be the first sign of cancer, particularly cancers of the pancreas, stomach, esophagus, or lung.

Fever is very common with cancer, but is more often seen in advanced disease. Almost all patients with cancer will have fever at some time, especially if the cancer or its treatment affects the immune system and makes it harder for the body to fight infection. Less often, fever may be an early sign of cancer, such as with leukemia or lymphoma.

Fatigue may be an important symptom as cancer progresses. It may happen early, though, in cancers such as with leukemia, or if the cancer is causing an ongoing loss of blood, as in some colon or stomach cancers.

Pain may be an early symptom with some cancers such as bone cancers or testicular cancer. But most often pain is a symptom of advanced disease.

Along with cancers of the skin (see next section), some internal cancers can cause skin signs that can be seen. These changes include the skin looking darker (hyperpigmentation), yellow (jaundice), or red (erythema); itching; or excessive hair growth.

Alternatively, or in addition, cancer subtypes present specific signs or symptoms. Changes in bowel habits or bladder function could indicate cancer. Long-term constipation, diarrhea, or a change in the size of the stool may be a sign of colon cancer. Pain with urination, blood in the urine, or a change in bladder function (such as more frequent or less frequent urination) could be related to bladder or prostate cancer.

Changes in skin condition or appearance of a new skin condition could indicate cancer. Skin cancers may bleed and look like sores that do not heal. A long-lasting sore in the mouth could be an oral cancer, especially in patients who smoke, chew tobacco, or frequently drink alcohol. Sores on the penis or vagina may either be signs of infection or an early cancer.

Unusual bleeding or discharge could indicate cancer. Unusual bleeding can happen in either early or advanced cancer. Blood in the sputum (phlegm) may be a sign of lung cancer. Blood in the stool (or a dark or black stool) could be a sign of colon or rectal cancer. Cancer of the cervix or the endometrium (lining of the uterus) can cause vaginal bleeding. Blood in the urine may be a sign of bladder or kidney cancer. A bloody discharge from the nipple may be a sign of breast cancer.

A thickening or lump in the breast or in other parts of the body could indicate the presence of a cancer. Many cancers can be felt through the skin, mostly in the breast, testicle, lymph nodes (glands), and the soft tissues of the body. A lump or thickening may be an early or late sign of cancer. Any lump or thickening could be indicative of cancer, especially if the formation is new or has grown in size.

Indigestion or trouble swallowing could indicate cancer. While these symptoms commonly have other causes, indigestion or swallowing problems may be a sign of cancer of the esophagus, stomach, or pharynx (throat).

Recent changes in a wart or mole could be indicative of cancer. Any wart, mole, or freckle that changes in colour, size, or shape, or loses its definite borders indicates the potential development of cancer. For example, the skin lesion may be a melanoma.

A persistent cough or hoarseness could be indicative of cancer. A cough that does not go away may be a sign of lung cancer. Hoarseness can be a sign of cancer of the larynx (voice box) or thyroid.

While the signs and symptoms listed above are the more common ones seen with cancer, there are many others that are less common and are not listed here. However, all art-recognized signs and symptoms of cancer are contemplated and encompassed herein.

The methods described herein encompass a variety of subjects, all of whom are mammals. The mammal may be a human, non-human primate, mousse, rat, dog, cat, horse, or cow, but are not limited to these examples. Mammals other than humans are advantageously used as subjects that represent animal models of a particular disorder. The preferred subject is human. A subject is male or female.

Subjects are identified as having a mutation in a miRNA binding site. Subjects having been identified with at least one mutation in a miRNA binding site, have not presented signs or symptoms of a disorder, such as cancer. Alternatively, subjects having been identified with at least one mutation in a miRNA binding site, have presented signs or symptoms of a disorder, such as cancer. Optionally, subjects have been diagnosed with one or more disorders, such as cancer. Subjects may have been diagnosed with cancers most frequently associated with the presence of the LCS6 SNP including, but not limited to, all varieties oflung cancer (e.g., non-small cell lung cancer (NSCLC) and small cell lung cancer), ovarian cancer, breast cancer, uterine cancer, head and neck cancer, pancreatic cancer, and colon cancer.

Subjects of the methods herein may not have any mutations in a mRNA binding site. Many of the genes that are overexpressed in cancer contain *let*-7 miRNA binding sites. As such, the modified oligos and compositions described herein, when administered to a subject lacking mutations, will repress such overexpression of let-7 target genes. The gene expression profiles of intended subjects may be analyzed and the appropriate modified oligos capable of silencing gene overexpression are administered. For example, a modified oligo (SEQ ID NOs: 22-36) that binds the LCS6 SNP containing KRAS allele, also binds a wild type KRAS allele and effectively decreases expression.

The disclosure provides a method of treating or alleviating a symptom of cancer by administering a composition comprising a modified oligo and at least one cytotoxic compound. Cytotoxic compounds include, but are not limited to, all forms of radioactive isotopes and chemotherapeutic compounds, e.g. chemotherapy drugs. Exemplary radioactive isotopes include, but are not limited to, molybdenum-99, technetium-99m, bismuth-213, carbon-11, chromium-51, cobalt-57, cobalt-60, copper-64, dysprosium-165, erbium-169, fluorine-18, gallium-67, holmium-166, indium-111, iodine-123, iodine-125, iodine-131, iridium-192, iron-59, krypton-81m, lutetium-177, nitrogen-13, oxygen-15, palladium-103, phosphorus-32, potassium-42, rhenium-186, rhenium-188, rubidium-81, samarium-153, selenium-75, sodium-24, strontium-89, strontium-92, thallium-201, xenon-133, ytterbium-177, ytterbium-169, yttrium-90, and radioisotopes of caesium, gold, and rthenium. Exemplary chemotherapy drugs include, but are not limited to, Dacarbazine/DTIC, Fluorouracil/5-FU, Fludarabine, Gemcitabine, Trastuzumab/Herceptin, Hydroxyurea/Hydrea, Idarubicin, Ifosfamide, Irinotecan, Cladribine/Leustatin, Mercaptopurine/Purinethol/6-MP, Methotrexate; Mithramycin/Plicamycin, Mitomycin, Mitoxanthrone/Novatrone, Navelbine/Vinorelbine, Nitrogen Mustard, Rituxan, Paclitaxel/Taxol, Docetaxel/Taxotere, Topotecan, Velban/Vinblastine, Vincristine, and Etoposide/VP-16.

### pharmaceutical Compositions

The disclosure provides a composition including at least one modified oligo and a pharmaceutically acceptable carrier. Pharmaceutically acceptable carriers are covalently or non-covalently bound, admixed, encapsulated, conjugated, operably-linked, or otherwise associated with the modified oligo such that the pharmaceutically acceptable carrier increases the cellular uptake, stability, solubility, half-life, binding efficacy, specificity, targeting, distribution, absorption, or renal clearance of the modified oligo molecule. Alternatively, or in addition, the pharmaceutically acceptable carrier increases or decreases the immunogenicity of the modified oligo molecule. Furthermore, the pharmaceutically acceptable carrier is capable to increasing the cytotoxicity of the modified oligo composition with respect to the targeted cancer cells.

Alternatively, or in addition, pharmaceutically acceptable carriers are salts (for example, acid addition salts, e.g., salts of hydrochloric, hydrobromic, acetic acid, and benzene sulfonic acid), esters, salts of such esters, or any other compound which, upon administration to a subject, are capable of providing (directly or indirectly) the biologically active compositions herein described. As such, the disclosure encompasses prodrugs, and other bioequivalents. As used herein, the term "prodrug" is meant to describe, a pharmacological substance that is administered in an inactive (or significantly less active) form. Once administered, the prodrug is metabolized *in vivo* into an active metabolite. Pharmaceutically acceptable carriers are alternatively or additionally diluents, excipients; adjuvants, emulsifiers; buffers, stabilizers, and/or preservatives.

Pharmaceutically acceptable carriers are modified oligo delivery systems/mechanisms that increase uptake of the modified oligo by targeted cells. For example, pharmaceutically acceptable carriers are viruses, recombinant viruses, engineered viruses, viral particles, replication-deficient viruses, liposomes, cationic lipids, anionic lipids, cationic polymers, polymers, hydrogels, micro- or nano-capsules (biodegradable), micropheres (optionally bioadhesive), cyclodextrins, plasmids, mammalian expression vectors, proteinaceous vectors, or any combination of the preceding elements (see, O'Hare and Normand, International PCT Publication No. WO 00/53722; U.S. Patent Publication 2008/0076701). Moreover, pharmaceutically acceptable carriers that increase cellular uptake can be modified with cell-specific proteins or other elements such as receptors, ligands, antibodies to specifically target cellular uptake to a chosen cell type.

Compositions may be first introduced into a cell or cell population that is subsequently administered to a subject. A modified oligo may be delivered intracellularly, e.g., in cells of a target tissue such as lung, or in inflamed tissues. Disclosed herein are compositions and methods for delivery of an isolated modified oligo and/or composition by removing cells of a subject, delivering the isolated modified oligo or composition to the removed cells, and reintroducing the cells into a subject. A modified oligo molecule may be combined with a cationic lipid or transfection material such as LIPOFECTAMINE (Invitrogen).

The active compounds may be prepared with pharmaceutically acceptable carriers that will protect the modified oligo molecule against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Examples of materials which can form hydrogels include polylactic acid, polyglycolic acid, PLGA polymers, alginates and alginate derivatives, gelatin, collagen, agarose, natural and synthetic polysaccharides, polyamino acids such as polypeptides particularly poly(lysine), polyesters such as polyhydroxybutyrate and poly-epsilon.-caprolactone, polyanhydrides; polyphosphazines, poly(vinyl alcohols), poly(alkylene oxides) particularly poly(ethylene oxides), poly(allylamines)(PAM), poly(acrylates), modified styrene polymers such as poly(4-aminomethylstyrene), pluronic polyols, polyoxamers, poly(uronic acids), poly(vinylpyrrolidone) and copolymers of the above, including graft copolymers.

Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

Pharmaceutically acceptable carriers are cationic lipids that are bound or associated with modified oligos. Alternatively, or in addition, modified oligos are encapsulated or surrounded in cationic lipids, e.g. lipsosomes, for in vivo delivery. Exemplary cationic lipids include, but are not limited to, N41-(2,3-dioleoyloxy)propyli-N,N,N-trimethylammonium chloride (DOTMA); 1,2-bis(oleoyloxy)-3-3-(trimethylammonium)propane (DOTAP), 1,2-bis(dimyrstoyloxy)-3-3-(trimethylammonia)propane (DMTAP); 1,2-dimyristyloxypropyl-3-dimethylhydroxyethylammonium bromide (DMRIE); dimethyldioctadecylammunium bromide (DDAB); 3-(N-(N',N'-dimethylaminoethane)carbamoyl)cholesterol (DC-Chol); 3.beta.-[N',N'-diguanidinoethyl-aminoethane)carbamoyl cholesterol (BGTC); 2-(2-(3-(bis(3-aminopropyl)amino)propylamino)acetamido)-'N,N-ditetradecylacetamide (RPR209120); pharmaceutically acceptable salts thereof, and mixtures thereof. Further examplary cationic lipids include, but are not limited to; 1,2-dialkenoyl-sn-glycero-3- ethylphosphocholines (EPCs), such as 1,2-dioleoyl-sn-glycero-3-ethylphosphocholine, 1,2-distearoyl-sn-glycero-3-ethylphosphocholine, 1,2-dipalmitoyl-sn-glycero-3-ethylphosphocholine, pharmaceutically acceptable salts thereof; and mixtures thereof.

Exemplary polycationic lipids includes but are not limited to, tetramethyltetrapalmitoyl spermine (TMTPS), tetramethyltetraoleyl spermine (TMTOS), tetrametblytetrilainyl spermine (TMTLS), tetramethyltetramyristyl spermine (TMTMS), tetramethyldioleyl spermine (TMDOS), pharmaceutically acceptable salts thereof, and mixtures thereof Further examplary polycationic lipids include, but are not limited to, 2,5-bis(3-aminopropylamino)-N-(2-((dioctadecylamino)-2-oxoethyl)pentanamid- e (DOGS); 2,5-bis(3-aminopropylamino)-N-(2-(di(Z)-octadeca-9-dionylamino)- 2- oxoethyl) pentanamicle (DOGS-9-en); 2,5-bis(33-aminopropylamino)-N-(2-(di(9Z,12Z)- octadeca-9,12-dienylamino)-2- -oxoethyl)pentanamide (DLinGS); 3-beta-(N.sup.4- (N.sup.1, N.sup.8-dicarbobenzoxyspermidine)carbamoyl)chole- sterol (GL-67); (9Z,9^{y}Z)- 2-(2,5=bis(3-atninopropylamino)pentanamido)pxopane-1;3-diyl-dioct- adec-9-enoate (DOSPER); 2,3-dioleyloxy-N-[2(sperminecarboxamido)ethlyl]-N,N-dimethyl-1-propanamini- um trifluoro-acetate (DOSPA); phamlaceutically acceptable salts thereof, and mixtures thereof.

Examples of cationic lipids are described in U.S. Pat. Nos. 4,897,355; 5,279,833; 6,733;777; 6,376,248; 5,736,392; 5,334,761; 5,459;127; 2005/0064595; U.S. Pat. Nos. 5,208,036; 5,264,618; 5,279,833; 5,283,185; 5,753,613; and 5,785,992.

Pharmaceutically acceptable carriers also include non-cationic lipids, such as neutral, zwitterionic, and anionic lipids. Examplary non-cationic lipids include, but are not limited to, 1,2-Dilauroyl-sn-glycerol (DLG); 1,2-Dimyristoyl-snglycerol (DMG); 1,2-Dipalmitoyl-sn-glycerol (DPG); 1,2-Distearoyl-sn-glycerol (DSG); 1,2-Dilauroyl-sn-glycero-3-phosphatidic acid (sodium salt; DLPA); 1,2-Dimyristoyl-snglycero-3-phosphatidic acid (sodium salt; DMPA); 1,2-Dipalmitoyl-sn-glycero-3-phosphatidic acid (sodium salt; DPPA); 1,2-Distearoyl-sn-glycero-3-phosphatidic acid (sodium salt; DSPA); 1,2-Diarachidoyl-sn-glycero-3-phosphocholine (DAPC); 1,2- Dilauroyl-sn-glycero-3-phosphocholine (DLPC); 1,2-Dimyristoyl-sn-glycero-3- phosphocholine (DMPC); 1,2-Dipalmitoyl-sn-glycero-0-ethyl-3-phosphocholine (chloride or triflate; DPePC); 1,2-Dipalmitoyl-sn-glycero-3-phosphocholine (DPPC); 1,2-Distcaroyl-sn-glycero-3-phosphocholine (DSPC); 1,2-Dilauroyl-sn-glyccro-3- phosphoethanolamine (DLPE); 1,2-Dimyristoyl-sn-glycero-3-phosphoethanolamine (DMPE); 1,2-Dipalmitoyl-sn-glycero-3-phosphoethanolamine (DPPE); 1,2-Distearoylsn-glycero-3-phosphoethanolamine (DSPE); 1,2-Dilauroyl-sn-glycero-3-phosphoglycerol (sodium salt; DLPG); 1,2-Dimyristoyl-sn-glycero-3-phosphoglycerol (sodium salt; DMPG); 1,2-Dimyristoyl-sn-glycero-3-phospho-sn-1-glycerol (ammonium salt; DMP-sn1-G); 1,2-Dipalmitoyl-sn-glycero-3-phosphoglycerol (sodium salt; DPPG); 1,2-Distearoyl-sn-glycero-3-phosphoglycero (sodium salt; DSPG); 1,2-Distearoyl-snglycero-3-phospho-sn-I-glycerol (sodium salt; DSP-sn-1-G); 1,2-Dipalmitoyl-snglycero-3-phospho-L-serine (sodium salt; DPP S); 1-Palmitoyl-2-linoleoyl-sn-glycero-3-phosphocholine (PLinoPC); 1-Palmitoy 1-2-oleoyl-sn-glycero-3-phosphocholine (POPC); 1-Palmitoy 1-2-oleoyl-sn-glycero-3-phosphoglycerol (sodium salt; POPG); 1-Palmitoyl-2-oleoyl-sn-glycero-3-phosphoglycerol (sodium salt; POPG); 1-Palmitoyl-2-oleoyl-snglycero-3-phosphoglycerol (ammonium salt; POPG); 1-Palmitoyl-2-4o-sn-glycero-3-phosphocholine (P-lyso-PC); 1-Stearoyl-2-lyso-sn-glycero-3-phosphocholine (S-lysoPC); and mixtures thereof Further examplary non-cationic lipids include, but are not limited to, polymeric compounds and polymer-lipid conjugates or polymeric lipids, such as pegylated lipids, including polyethyleneglycols, N-(Carbonyl-methoxypolyethyleneglycol-2000)-1,2-dimyristoyl-sn-glycero-3-phosphoethanolamine (sodium salt; DMPE-MPEG-2000); N-(Carbonyl-methoxypolyethyleneglycol-5000)-1,2-dimyristoyl-sn-glycero-3-- phosphoethanolamine (sodium salt; DMPE-MPEG-5000); N-(Carbonyl-methoxypolyethyleneglycol 2000)-1,2-dipalmitoyl-sn-glycero-3-phosphoethanolamine (sodium salt; DPPE-MPEG-2000); N-(Carbonyl-methoxypolyethyleneglycol 5000)-1,2-dipalmitoyl-sn-glycero-3-phosphoethanolamine (sodium salt; DPPE-MPEG-5000); N-(Carbonyl-methoxypolyethyleneglycol 750)-1,2-distearoyl-sn-glycero-3-phosphoethanolamine (sodium salt; DSPE-MPEG-750); N-(Carbonyl-methoxypolyethyleneglycol 2000)-1,2-distearoyl-sn-glycero-3-phosphoethanolamine (sodium salt; DSPE-MPEG-2000); N-(Carbonyl-methoxypolyethyleneglycol 5000)-1,2-distearoyl-sn-glycero-3-phosphoethanolamine (sodium salt; DSPE-MPEG-5000); sodium cholesteryl sulfate (SCS); pharmaceutically acceptable salts thereof, and mixtures thereof Examples of non-cationic lipids include, but are not limited to, dioleoylphosphatidylethanolamine (DOPE), diphylanoylphosphatidylethanolamine (DPhPE), 1,2-Diolcoyl-sn-Glycero-3-Phosphocholine (DOPC), 1,2-Diphylanoyl-sn-Glycero-3-Phosphocholine (DPhPC), cholesterol, and mixtures thereof.

Pharmaceutically-acceptable carriers further include anionic lipids. Examplary anionic lipids include, but are not limited to, phosphatidylserine, phosphatidic acid, phosphatidylcholine, platelet-activation factor (PAF), phosphatidylethanolamine, phosphatidyl-DL-glycerol, phosphatidylinositol, phosphatidylinositol (pi(4)p, pi(4,5)p2), cardiolipin (sodium salt), lysophosphatides, hydrogenated phospholipids, sphingoplipids, gangliosides, phytosphingosine, sphinganines, pharmaceutically acceptable salts thereof, and mixtures thereof.

Supplemental or complementary methods for delivery of nucleic acid molecules for use herein are described, e.g:, in Akhtar, et al., Trends Cell Bio. 2:139, 1992; Delivery Strategies for Antisense Oligonucleotide Therapeutics, ed. Akhtar, 1095; Maurer, et al., Mol. Membr. Biol. 16:129-140, 1999; Hofland and Hung, Handb. Exp. Pharmacol. 137:165-192, 1999; and Lee, et al., ACS Symp. Ser. 752:184-1.92, 2000. Sullivan, et al., International PCT Publication No. WO 94/02595, further describes general methods for delivery of enzymatic nucleic acid molecules. These protocols can be utilized to supplement or complement delivery of virtually any nucleic acid molecule described herein.

Pharmaceutical compositions are administered locally and/or systemically. As used herein, the term "local administration" is meant to describe the administration of a pharmaceutical composition describe herein to a specific tissue or area of the body with minimal dissemination of the composition to surrounding tissues or areas. Locally administered pharmaceutical compositions are not detectable in the general blood stream when sampled at a site not immediate adjacent or subjacent to the site of administration.

As used herein the term "systemic administration" is meant to describe in vivo systemic absorption or accumulation of drugs in the blood stream followed by distribution throughout the entire body. Administration routes which lead to systemic absorption include, without limitation: intravenous, subcutaneous, intraperitoneal, inhalation, oral, intrapulmonary and intramuscular. Each of these administration routes exposes the desired negatively charged polymers, e.g., nucleic acids, to an accessible diseased tissue. The rate of entry of a drug into the circulation has been shown to be a function of molecular weight or size. The use of a liposome or other drug carrier comprising the compounds of the instant disclosure can potentially localize the drug, e.g., in certain tissue types, such as the tissues of the reticular endothelial system (RES). A liposome formulation that can facilitate the association of drug with the surface of cells, such as, lymphocytes and macrophages is also useful. This approach may provide enhanced delivery of the drug to target cells by taking advantage of the specificity of macrophage and lymphocyte immune recognition of abnormal cells, such as cancer cells.

A pharmaceutically acceptable carrier is chosen to be compatible with its intended route of administration. Examples of routes of administration include parenteral, e.g., intravenous, intradermal, subcutaneous, oral (e,g., inhalation or insufflation), transdermal (topical), transmucosal, transopthalmic, tracheal, intranasal, epidermal, intraperitoneal, intraorbital, intraarterial, intracapsular, intraspinal, intrastemal, intracranial, intrathecal, intraventricular, and rectal administration. Alternatively, or in addition, compositions of the disclosure are administered non-parentally, for example, orally. Alternatively, or further in addition, compositions described herein are administered surgically, for example, as implants or biocompatible polymers.

Pharmaceutical compositions are administered via injection or infusion, e.g. by use of an infusion pump. Direct injection of the nucleic acid molecules described herein, is performed using standard needle and syringe methodologies, or by needle-free technologies such as those described in Corny et al., Clin. Cancer Res. 5:2330-2337, 1999 and Barry et al., International PCT Publication No. WO 99/31262.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

An isolated nucleic acid with a pharmaceutically acceptable carrier can be administered to a subject in many of the well-known methods currently used for chemotherapeutic treatment. For example, for treatment of cancers, a compound described herein may be injected directly into tumors, injected into the blood stream or body cavities or taken orally or applied through the skin with patches. The dose chosen should be sufficient to constitute effective treatment but not so high as to cause unacceptable side effects. The state of the disease condition (e.g., cancer, precancer, and the like) and the health of the subject should preferably be closely monitored during and for a reasonable period after treatment.

Compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof.

The pharmaceutical compositions are in the form of a sterile injectable aqueous or oleaginous suspension. This suspension is formulated according to the known art using those suitable dispersing or wetting agents and suspending agents that have been mentioned above. The sterile injectable preparation is a sterile injectable solution or suspension in a non-toxic parentally acceptable diluent or solvent, e.g., as a solution in 1,3-butanediol. Exemplary acceptable vehicles and solvents are water, Ringer's solution and isotonic sodium chloride solution. In addition, sterile, fixed oils are conventionally employed as a solvent or suspending medium. For this purpose, any bland fixed oil is employed including synthetic mono-or diglycerides. In addition, fatty acids such as oleic acid are used in the preparation of injectables.

Sterile injectable solutions can be prepared by incorporating the modified oligo in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible pharmaceutically acceptable carrier. Modified oligos containing at least one 2'-0-methoxyethyl modification are used when formulating compositions for oral administration. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide, a sweetening agent such as sucrose or saccharin; or a flavoring agent such as peppermint, methyl salicylate, or orange flavoring.
For administration by inhalation, the compounds are delivered in the form of an aerosol spray from pressured container or dispenser, which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Exemplary penetrants for transdermal administration include, but are not limited to, lipids, liposomes, fatty acids, fatty acid, esters, steroids, chelating agents, and surfactants. Preferred lipids and liposomes are neutral, negative, or cationic. Compositions are encapsulated within liposomes or form complexes thereto, such as cationic liposomes.

Alternatively, or in addition, compositions are complexed to lipids, such as cationic lipids. Compositions prepared for transdermal administration are provided by iontophoresis. Such penetrants are generally known in the art, and include, for example, for transmucosal administration, detergents, bile salts, and fusidic acid derivatives.

Transmucosal administration can be accomplished through the use of nasal sprays or suppositories. For transdermal administration, the active compounds are formulated into patches, ointments, lotions, salves, gels, drops, sprays, liquids, powder, or creams as generally known in the art.

Pharmaceutical compositions are administered systemically and are intended to cross the blood-brain barrier to contact cells of the central nervous system. Alternatively, or in addition, Pharmaceutical compositions are administered intraspinally by, for example, lumbar puncture, or intracranially, e.g. intrathecally or intraventricularly. By the preceding routes, pharmaceutical compositions are introduced directly into the cerebral spinal fluid. Nonlimiting examples of agents suitable for formulation with the nucleic acid molecules described herein, particularly for targeting nervous system tissues, include: P-glycoprotein inhibitors (such as Pluronic P85), which can enhance entry of drugs into the GNS (Jolliet-Riant and Tillement, Fundam. Clin. Pharamacol. 13:16-26, 1999); biodegradable polymers, such as poly (DL-lactide-coglycolide) microspheres for sustained release delivery after intracerebral implantation (Emerich, D. F., et al., Cell Transplant 8:47-58, 1999) (Alkermes, Inc. Cambridge, Mass.); and loaded nanoparticles, such as those made of polybutyleyanoacrylate, which can deliver drugs across the blood brain barrier and can alter neuronal uptake mechanisms (Prog. Neuropsychopharmacol Biol. Psychiatry 23:941-949, 1999). Other non-limiting examples of delivery strategies for the nucleic acid molecules of the instant disclosure include material described in Boado, et al., J. Pharm. Sci. 87:1308-1315,1998; Tyler, et al., FEBS Lett. 421:280-284,1999; Pardridge, et al, PNAS USA. 92:5592-5596, 1995; Boado, Adv. Drug Delivery Rev. 15:73-107, 1995; Aldrian-Herrada, et al., Nucleic Acids Res. 26:4910-49916, 1998; and Tyler, et al., PNAS USA. 96:7053-7058, 1999.

The modified oligos and compositions described herein are also administered in the form of suppositories, e.g., for rectal administration of the drug. These compositions are prepared by mixing the drug with a suitable non-irritating excipient that is solid at ordinary temperatures but liquid at the rectal temperature and will therefore melt in the rectum to release the drug. Such materials include cocoa butter and polyethylene glycols.

Aqueous suspensions contain the active materials in admixture with excipients suitable for the manufacture of aqueous suspensions. Such excipients are suspending agents, e.g., sodium carboxymethylcellulose, methylcellulose, hydropropylmethylcellulose; sodium alginate, polyvinylpyrrolidone, gum tragacanth and gum acacia; dispersing or wetting agents can be a naturally-occurring phosphatide, e.g., lecithin, or condensation products of an alkylene oxide with fatty acids, e.g., polyoxyethylene stearate, or condensation products of ethylene oxide with long chain aliphatic alcohols, e.g., heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, e.g., polyethylene sorbitan monooleate. The aqueous suspensions also contain one or more preservatives, e:g., ethyl, or n-propyl phydroxybenzoate, one or more coloring agents, one or more flavoring agents, and one or more sweetening agents, such as sucrose or saccharin.

Oily suspensions are formulated by suspending the active ingredients in a vegetable oil, e.g., arachis oil, olive oil, sesame oil or coconut oil, or in a mineral oil such as liquid paraffin. The oily suspensions contain a thickening agent, e.g., beeswax, hard paraffin or cetyl alcohol. Sweetening agents and flavoring agents are added to provide palatable oral preparations. These compositions are preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, e.g., sweetening, flavoring and coloring agents, are also present.

Pharmaceutical compositions described herein are in the form of oil-in-water emulsions. The oily phase is a vegetable oil or a mineral oil or mixtures of these. Suitably emulsifying agents are naturally-occurring gums, e.g., gum acacia or gum tragacanth, naturally-occurring phosphatides, e.g., soy bean, lecithin, and esters or partial esters derived from fatty acids and hexitol, anhydrides, e.g., sorbitan monooleate, and condensation products of the said partial esters with ethylene oxide, e.g., polyoxyethylene sorbitan monooleate. The emulsions also contain sweetening and flavoring agents.

Preferably, the pharmaceutically acceptable carrier can be a solubilizing carrier molecule. More preferably, the solubitizing carrier molecule can be Poloxamer, Povidone K17, Povidone K12, Tween 80, ethanol, Cremophor/ethanol, Lipiodol, polyethylene glycol (PEG) 400, propylene glycol, Trappsol, alpha-cyclodextrin or analogs thereof, beta-cyclodextrin or analogs thereof, and gamma-cyclodextrin or analogs thereof.

The disclosure also provides compositions prepared for storage or administration. Acceptable carriers or diluents for therapeutic use are well known in the pharmaceutical art, and are described, e.g., in Remington's Pharmaceutical Sciences, Mack Publishing Co., A. R. Gennaro Ed., 1985. For example, preservatives, stabilizers, dyes and flavoring agents are provided. These include sodium benzoate, sorbic acid and esters of phydroxybenzoic acid. In addition, antioxidants and suspending agents are used.

### EXAMPLES

### Example 1: Clonogenic Assays to Measure Targeting SNPs

Cancer cells and/or cell lines of interest are transfected with targeted and control oligonucleotide, double-stranded RNA (dsRNA), and DNA molecules designed to target an identified single nucleotide polymorphism (SNP). Experiments involving cancer cells and/or cell lines that carry a mutation, often a SNP, with identical or similar cancer cells and/or cell lines that do not carry the mutation are often conducted in parallel.

The transfection method used was optimized by using a luciferase (luc) reporter construct sensitive to microRNA levels *(luc.* fused to the *NRAS* 3'UTR, see NCBI Accession No. NM_ 002524 for NRAS sequence). The chosen transfection method for these studies causes the least toxicity and leads to the most efficient transfection (X-tremeGENE, Roche, data not shown Following transfection, cells are plated at a range of dilutions and grown without being disturbed for 2 weeks in order to allow for colony formation. Colony counts are performed by using cell staining procedures. The appearance of a colony represents the survival of a cell and it's clonal progeny. As such, the total amount of cell survival can be represented by the number of colonies formed as a result of each treatment.

In cases where targeting of the SNP by a nucleic acid is tested in combination with cytotoxic treatment. (such as radiation or chemotherapy), cells are treated 24 hours post-transfection with increasing doses of cytotoxic treatment and then plated at a range of dilutions and grown without being disturbed. Cell survival is assessed as described *supra.* Experiments are performed in quadruplicate for each dose and for each SNP targeting method. Experiments are repeated at a minimum of two times. Stratified t-tests are performed to analyze statistical significance for experiments.

### Example 2: Direct Targeting of Modified Oligos to the KRAS 3'UTR: Design of RNA-DNA Chimeras.

Modified oligos, also referred to herein as siRNA, consisting of, consisting essentially of, or comprising double stranded RNA-DNA oligomer chimeras were designed to target the LCS6 SNP. The use of RNA-DNA chimeras, as opposed to double stranded RNA-RNA chimeras, minimizes off-targeting for a number of reasons including, but not limited to, inhibiting introduction of the passenger strand (PS) into the RNA-Induced Silencing Complex (RISC), rendering the PS non-functional, and making the seed binding more specific.

The use of RNA-DNA chimeras inhibits introduction of the PS into the RISC through altered binding energies between the two strands. PNA binding to DNA (and DNA binding to DNA) shows a lower binding energy than RNA binding to RNA. The strand with the weaker binding at the 5'end is incorporated into the RISC, therefore introducing DNA (or mismatches) at the 5' end of the guide strand (GS) introduces a bias for incorporating the guide strand over the passenger strand. A bias for incorporation of the GS over the PS not only prevents off-targeting but also makes the siRNA more efficient.

The use of RNA-DNA chimeras renders the PS non-functional. SiRNAs with DNA bases at the 3'end are non-functional. Thus introducing DNA bases at the 3'end of the PS avoids off-target effects.

The use of RNA-DNA chimeras makes the seed binding more specific. As a result of the lower binding energy between DNA and RNA, siRNA with. DNA bases in the seed region (typically the first 2-10 nucleotides of the 5'end) show higher sensitivity to mismatches in the seed region when binding to target mRNA. Thus, siRNAs with DNA bases incorporated into the seed region decrease or minimize off-targeting.
Alternatively, or in addition, modified bases like 2'OM are added into the seed region in order to further reduce off-targeting.

### Example 3: Direct Targeting of Modified Oligos to the KRAS 3'UTR: Generation of Modified Oligo 1.

Methods disclosed herein include directly targeting modified oligo molecules to *let-7* complementary sites (LCSs) with the 3'UTRs of genes such as KRAS.

The targeted gene may be human KRAS (known by gene ID *Hs KRAS*, and GenBank Accession No. M54968. Within this human KRAS gene, the LCS6 SNP occurs at nucleotide base pair 2509. Modified oligos herein described not only target miRNA binding sites, but also any region or sequence that includes, contains, or comprises the LCS6 SNP. Modified oligos, for example, those provided herein are nonlimiting exemplary modified oligos. All modified oligos that specifically target a region or sequence that includes, contains, or comprises the LCS6 SNP are encompassed by the disclosure.

The following sequences relate to modified oligo 1 wherein RNA are in black small letters, DNA are in capital letters, and sequence modifications in bold. Guide Strands (GS) are paired with Passenger Strands (PS) to form double-stranded siRNA molecules. Degrees are Celcius. The "G" within the targeted sequence represents the LCS6 SNP.

| | | |
|---|---|---|
| Targeted Sequence | | cctgacctcaagtgatGcacc (SEQ ID NO: 23) |
| | GS1 | **T**GTGCATCacuugaggucagg (SEQ ID NO: 24) |
| | GS2 | **u**gugcaucacuugaggucagg (SEQ ID NO: 25) |
| | GS3 | ggugcaucacuugaggucagg (SEQ ID NO: 26) |
| | PS | UgaccucaagugaTGCACCCA (SEQ ID NO: 27) |

Melting temperatures 8 nucleotides 5' GS:
RNA-RNA -> 36.8 deg
RNA-DNA -> 20.8 deg
DNA-DNA -> 21.5 deg

Melting temperature 8 nucleotides 5' PS:
RNA-RNA -> 36.9 deg

Melting temperature 8 nucleotides 5' GS with mismatch:
RNA-DNA -> < 10 deg
DNA-DNA -> 10.9 deg

### Example 4: Direct Targeting of Modified Oligos to the KRAS 3'UTR: Generation of Modified Oligo 2.

The following sequences relate to modified oligo 2 wherein RNA are in black small letters, DNA are in capital letters, and sequence modifications in bold. Guide Strands (GS) are paired with Passenger Strands (PS) to form double-stranded siRNA molecules. Degrees are Celsius. The "G" within the targeted sequence represents the LCS6 SNP.

| | | |
|---|---|---|
| Targeted Sequence | | ctcctgacctcaagtgatGca (SEQ ID NO: 28) |
| | GS1 | ugcaucacuugaggucaggag (SEQ ID NO: 29) |
| | GS2 | TGCATCACuugaggucaggag (SEQ ID NO: 30) |
| | PS1 | ccugaccucaagugaugcacc (SEQ ID NO: 31) |
| | PS2 | ccugaccucaaguGATGCACC (SEQ ID NO: 32) |

Melting temperature 8 nucleotides 5' GS:
RNA-RNA -> 32.0 deg
RNA-DNA -> 12.5 deg
DNA-DNA -> 18.4 deg

Melting temperature 8 nucleotides 5' PS:
RNA-RNA -> 40.7 deg

### Example 5: Direct Targeting of Modified Oligos to the KRAS 3'UTR: Generation of Modifed Oligo 3.

The following sequences relate to modified oligo 3 wherein RNA are in black small letters, DNA are in capital letters, and sequence modifications in bold. Guide Strands (GS) are paired with Passenger Strands (PS) to form double-stranded siRNA molecules. Degrees arc Celsius. The "G" within the targeted sequence represents the LCS6 SNP.

| | | |
|---|---|---|
| Targeted sequence | | actcctgacctcaagtgatGc (SEQ ID NO: 33) |
| | GS | **u**caucacuugaggucaggagu (SEQ ID NO: 34) |
| | PS1 | uccugaccucaagTGATGCAC (SEQ 1D NO: 35) |
| | PS2 | uccugaccucaagugaugcac (SEQ ID NO: 36) |

Melting temperature 8 nucleotides 5' GS:
RNA-RNA -> 16.3 deg

Melting temperature 8 nucleotides 5' PS:
RNA-RNA -> 41.5 deg

### Example 6: Direct Targeting of Modified Oligos to the KRAS 3'UTR: Luciferase Assay

The modified oligos described in Examples 3-5 were resuspended and then annealed (combinations are detailed below).

Combination of Annealed Oligos Used (see, Figures 2B, 3, 4, and 5):
KRAS #1-1 KRAS1 GS1 + KRAS1 PS
KRAS #1-2: KRAS1 Gas2 + KRAS1 PS
KRAS #1-3: KRAS1 GS3 + KRAS1 PS
KRAS #2-1: KRAS2 GS1 + KRAS2 PS1
KRAS #2-2: KRAS2 GS + KRAS2 PS2
KRAS #2-3: KRAS2 GS2 + KRAS2 PS1
KRAS #2-4: KRAS2 GS2 + KRAS2 PS2
KRAS #3-1: KRAS3 GS1 +KRAS3 PS1
KRAS #3-2: KRAS3 GS1 + KRAS3 PS2

Annealed modified oligos were transfected into 293T cells using Lipofectamine™. Luciferase reporter constructs containing the KRAS 3'UTR, either with or without the LCS6 SNP, were also transfected into these 293T cells. Annealed modified oligos bind to either luciferase construct and silence gene expression by either degradation of the reporter protein construct or inhibition of translation of the reporter protein construct.

The negative control used for these luciferase assays was Qiagen AllStars negative Control siRNA with no modifications. This negative control has been checked for off targeting by microarray and has been experimentally validated for having no effect on cell cycle, viability, and/or nucleus size.

Firefly luciferase values were normalized to renilla luciferase values. The average of this ratio for the three independent transfections was calculated at each time point for each experiment. This calculated average was then normalized to the average ratio in the control siRNA transfection (Figure 2A and B):

Results of the luciferase reporter assay show that the Annealed modified oligos directly bind the KRAS 3'UTR containing the LCS6 SNP. Moreover, the annealed modified oligos are capable of specifically silencing the reporter construct containing the LCS6 SNP demonstrated by decreased luciferase expression from this construct compared to the wild type KRAS construct (Figure 2B). This assay is a proof-of-concept result showing that modified oligos can be engineered which directly target regions containing at least one SNP, e.g. the LCS6 SNP, and result in the silencing of the gene which contains this region.

### Example 7: Modified Oligo Treatment Decreases Survival of Multiple Cancer Cell Types

Different combinations of oligonucleotides were annealed and transfected into onco-SNP (LCS6 SNP) positive cell lines IGR-OV1 (ovarian), DU-145 (prostate), 789-0 (renal), MIAPACA (pancreatic), EKVX (lung) and MCF7 (breast) (*see*, Example 6, paragraph 176, for delineation of combinations). Cells were grown in clonogenic assays to determine the affect of the oligos on cell survival (*see*, Example 1 for clonogenic assay explanation).

Figure 3 shows that administration of one or more modified oligos leads to a decrease of cancer cell survival when that cancer cell contains the LCS6 SNP. The most dramatic results were observed with the ovarian, lung, breast, and pancreatic cancer cells lines. While more varied, effective cell death was observed in the prostate and renal cell lines when several of the oligonucleotide combinations were used. Thus, the data of Figure 3 show that administration of the modified oligonucleotides are an effective broad-spectrum treatment for cancers containing the LCS6 SNP.

The variation in efficacy between oligonucleotide combinations within a single tumor type can be explained in a number of ways. First, these oligonucleotide combinations contain different RNA to DNA ratios. As explained in Example 2, the use of RNA-DNA chimeras, as opposed to double-stranded RNA chimeras, can minimize off-targeting by a plurality of mechanisms, such as inhibiting introduction of the passenger strand (PS) into the RISC and rendering the PS non-functional during miRNA processing, as well as increasing the specificity of the seed sequence binding of the miRNA to the mRNA target (which lowers the binding energy between the mRNA and its RNA making the interaction more favorable and more probable). Second, as opposed to the binding events demonstrated in Figure 2B, in which the modified oligonucleotide targeted a synthetic and exogenously introduced construct containing the 3'UTR of KRAS fused to a luciferase gene; the oligonucleotides of Figure 3 target an endogenous KRAS mRNA. This second difference is significant because endogenous mRNAs have secondary structure that affects the availability of miRNA binding sites and the binding energies required for miRNA-mRNA interactions. Moreover, mRNAs are expressed at physiological levels that vary between cell lines, even those originating from the same tissue. mRNAs are also targeted by a variety of endogenous regulatory RNAs and proteins that compete with the modified oligonucleotide.

The cell lines used in Figure 3 were chosen not only because they contain the LCS6 SNP, but also because they are art-recognized models of these cancer types. However, the ordinarily skilled artisan would readily recognize that a cell line, particularly one which has been maintained in vitro for many generations, even though it contains the LCS6 SNP and overexpresses KRAS, may not be dependent upon KRAS for its oncogenic capabilities. Thus, even if the modified oligonucleotides effectively silenced KRAS in the cell lines of Figure 3, the cell survival may not have been effected in all clones. Thus, the efficacy of modified oligonucleotides is verified in primary tumour cells.

To further explain the individual variation, the LCS6 SNP cancer cell lines may express varying levels of endogenous let-7 miRNAs or varying alleles of let-7 miRNAs that compete with the modified oligonucleotides for binding the LCS6 SNP site. Although it is expected that an endogenous let-7 miRNA should be less efficacious at binding to the LCS6 SNP, it is possible that the sequences of some modified oligonucleotides allow far more favorable binding interactions and enable these oligos to better out-compete endogenous and ineffective miRNAs. To overcome this competition, the dosage of the modified oligonucleotide is increased. Alternatively, or in addition, more than one modified oligonucleotide is administered simultaneous or sequentially to a subject until a therapeutically favorable result is achieved.

Finally, any given modified oligonucleotide could have a sequence that is substantially similar to the mRNA target of an endogenous miRNA, and therefore, could be targeted by the host cell for degradation. To overcome this occurrence, either another modified oligonucleotide having a different sequence that also binds the LCS6 SNP is simultaneously or sequentially administered to the subject.

Critically, the data of Figure 3 are robust and demonstrate that the modified oligonucleotide combinations effectively decreased cell survival of each LCS6 SNP containing cancer cell type, overcoming each of the above obstacles, which are plausible and may explain the few outliers. Furthermore, because Figure 3 demonstrates that in every cancer cell type containing the LCS6 SNP, at least one, and in fact, multiple modified oligonucleotides effectively decreased cancer cell survival. Thus, administration of multiple modified oligonucleotides should ensure a positive therapeutic result provided the data of Figure 3.

The efficacy of administration of a composition containing a modified oligonucleotide to a cell is determined by comparing the efficacy of decreasing cancer cell survival of those cells, either LCS6-SNP positive or -negative, which received the modified oligo to those cells, either LCS6-SNP positive or -negative, which either received a negative oligonucleotide control oligo or a placebo negative control. An exemplary negative oligonucleotide control oligo is the Allstars negative control oligo, which is an annealed double stranded miRNA, available from Qiagen.

### Example 8: Modified Oligo Treatment Decreases Survival of Ovarian and Pancreatic Cancer Cell Types

Different combinations of oligonucleotides were annealed and transfected into onco-SNP (LCS6 SNP) positive ovarian and pancreatic cell lines (*see*, Example 6, paragraph 176, for delineation of combinations). Cells were grown in clonogenic assays to determine the affect of the oligos on cell survival (*see*, Example 1 for clonogenic assay explanation).

Figure 4 shows that administration of one or more modified oligos leads to a decrease of cancer cell survival when that cancer cell contains the LCS6 SNP, compared to cancer cells that do not contain the LCS6 SNP. Similar to Figure 3, and with very few exceptions, modified oligonucleotides effectively decreased cell survival of ovarian and pancreatic cancer cells when those cells contained the LCS6 SNP. Importantly, in each cancer type, Figure 4 shows one oligonucleotide combination which decreased cancer cell survival by at least 50%. Ovarian cancer cell survival decreased by more 50% following the administration of oligonucleotide combinations 1.3 and 2.1. Similarly, pancreatic cancer cell survival decreased by at least 50% following the administration of oligonucleotide combination 2.3.

### Example 9: Modified Oligo treatment Decreases Survival of Ovarian Cancer Cells

Different combinations of oligonucleotides were annealed and transfected into an onco-SNP (LCS6 SNP) positive ovarian cell line and an onco-SNP negative cell line (*see*, Example 6, paragraph 176, for delineation of combinations). Cells were grown in clonogenic assays to determine the affect of the oligos on cell survival (*see*, Example 1 for clonogenic assay explanation).

Figure 5 shows that administration of one or more modified oligonucleotides leads to decreased cancer cell survival when that cancer cell contained the LCS6 SNP, compared to cancer cells that did not contain the LCS6 SNP. All of the modified oligonucleotide combinations shown effectively decreased LCS6 SNP positive cancer cell survival compared to the SNP-negative ovarian cancer cell line. Similar to Figure 4, modified oligonucleotide combinations 1.3 and 2.1 decreased ovarian cancer cell survival by more than 50%.

### Example 10: Computational Prediction of Modified Oligonucleotide Binding

To determine whether modified oligonucleotides bind the LCS6 SNP, or another oncogene, and what amount of binding energy is required for the interaction to occur, a computational approach is used. For instance, a RNAhybrid, a publicly-available algorithm, is used to compare the binding energy of a modified oligonucleotide and a known miRNA to a given target RNA (see, Kruger, J. and Rehmsmeier, M. Nucleic Acids Research, 2006, 34:W451-454; Rehmsmeier, M. and Steffen, P. RNA, 2004, 10:1507-1517). One advantage of the RNAhybrid program is that parameters such as the length of the seed sequence, G:U base pairing, and a seed-match option can be modified to account for non-canonical interactions.

Thus, to use the above algorithm for the treatment of a subject, one or more KRAS alleles of a cancer subject is amplified (for instance, using polymerase chain reaction, PCR), particularly within the 3'UTR, and this portion of the amplified gene is sequenced according to methods known in the art. Once the DNA sequence of the KRAS gene is determined, the mRNA sequence is delineated according to the following rules: adenosine (DNA) pairs with uracil (RNA), thymine (DNA) pairs with adenosine (RNA), cytosine (DNA) pairs with guanine (mRNA), and guanine (DNA) pairs with cytosine (RNA). The mRNA sequence of the KRAS transcript is compared to the sequence of a modified oligonucleotide using RNAhybrid. If the binding energy required for the modified oligonucleotide to bind to the KRAS gene is less than the binding energy for a wild type or endogenous let-7 miRNA to bind to the same KRAS gene (this interaction is used as a control), then the modified oligonucleotide therapeutically silences KRAS. It is expected when a modified oligonucleotide has a more favorable binding energy than a wild type let-7 miRNA, the KRAS gene contains a mutation. That mutation is most often the LCS6 SNP.

### Example 11: Experimental Validation of Modified Oligonucleotide Efficacy in Primary Tumor Cells

In order to determine the effectiveness of a modified oligonucleotide in treating the cancer or tumor of a subject, a biopsy is performed, and primary tumor cells are removed. These *ex vivo* primary tumor cells, either taken from the same patient for which treatment is intended, or from patient of similar medical background, are tested for the presence or absence of the LCS6 SNP. Although it is expected that treatment with a modified oligonucleotide is more efficacious in those subjects who carry the LCS6 SNP mutation, compositions are intended for all KRAS- or RAS-dependent tumors. The term "similar medical background" is meant to describe a subject of equivalent age, gender, height, weight, medical history (e.g. history of medical conditions), LCS6 SNP status, cancer type and stage, and treatment-regime.

Alternatively, or in addition, combinations of modified oligonucleotides are annealed and transfected into primary tumor cells isolated from leukemia, lymphoma, carcinoma, sarcoma, germ cell cancers, or blastoma cancers (*see*, Example 6, paragraph 176, for exemplary combinations). Cells are grown in clonogenic assays to determine the affect of the modified oligos on cell survival (*see*, Example 1 for clonogenic assay explanation). Treated primary cancer cells are compared to untreated LCS6-SNP positive, treated LSC6-SNP negative, and/or untreated LCS6-SNP negative primary cancer cells.

Subjects whose primary cancer cells are responsive to modified oligonucleotides, e.g. primary cancer cells show decreased cell survival when compared to non-SNP containing primary cancer cells or to primary cells that are not treated with a composition containing a modified oligonucleotide, are administered a composition including at least one modified oligonucleotide, provided locally or systemically. Most often the composition administered to the subject contains the same modified oligonucleotide administered to the primary cancer cells. Optionally, a composition containing more than one modified oligonucleotide is used in the primary cancer cell test as well as the treatment of the subject.

### Example 12: Experimental Validation of Modified Oligonucleotide Efficacy and Delivery in Mouse Xenogtaft Models

An established human cancer cell line, as described in Example 7, or primary human cancer cells, as described in Example 11, is introduced into an immune-compromised mouse tumor model to determine the efficacy of treatment and delivery of modified oligonucleotide compositions in vivo. For instance, a mouse with decreased numbers of B-, T-, or Natural Killer (NK)- cells compared to a wild type mouse is considered immunocompromised. Exemplary immunocompromised mice commonly used for xenograft experiments include, but are not limited to, SCID, NOD, NSG (NOD SCID gamma), and Nude athymic mice. Additional examples can be found, for instance, at Jackson Laboratories' listing of mouse strains used as research tools for cancer (http://jaxmice.jax.org/list/rax3.html).

Xenograft mouse models are implanted with cancer cell lines or primary cancer cells which, optionally, carry the LCS6 SNP mutation and respond to modified oligonucleotide treatment in vitro. Cells are implanted either subcutaneously or orthotopically, e.g. at the site of the tumor origin to recapitulate the original human condition. Xenografts are either treated with a composition herein described or a control treatment (for instance, an antisense or scrambled oligonucleotide) and evaluated for changes in a variety of parameters including, but not limited to, tumor grade, tumor size, tumor encapsulation, metastatic potential or metastasis, tumor regression, cancer remission, tumor vascularization, tumor cell-mediated secretion of angiogenic or growth factors, tumor cell-mediated degradation of cellular matrix surrounding the tumor, and cancer cell survival or death. Exemplary signs of therapeutic effectiveness include improvements in tumor grade; decreases in tumor size; maintenance of tumor encapsulation; maintenance or decrease of metastatic potential; prevention, inhibition, or suspension of metastasis; maintenance or increased in tumor regression; maintenance of cancer remission; prevention, inhibition, or reversal of tumor vascularization; prevention or inhibition of tumor cell-mediated secretion of angiogenic or growth factors; prevention or inhibition of tumor cell-mediated degradation of cellular matrix; decrease in cancer cell survival; increase in cancer cell death. Moreover, prolonged survival of the mouse is also a sign of therapeutic efficacy.

Mice containing xenographs are monitored for signs of toxicity following treatment. Dosages of compositions are increased until the maximal tolerable amount is determined. In other terms, the no observed adverse effect levels (NOAELs) are determined. These dosages levels are translated using the United States Federal Drug Administration's guidelines to determine the Human Equivalent Dosage as a maximal recommended starting dose (MRSD) for human clinical trials. This conversion is typically based upon body surface area when a composition is administered systemically. However, when a composition is administered by a route for which the dose is limited to local toxicity (e.g., topical, intranasal, subcutaneous, intramuscular) the human concentration is normalized to concentration or amount of composition at the application site. Moreover, when the composition is administered to an anatomical compartment with little subsequent systemic distribution (e.g. intrathecal, intravesical, intraocular, intrapleural, and intraperitoneal), the human concentration should be normalized to the compartmental volumes and concentrations of the composition.

Preferably, the therapeutically effective dose of the composition is administered to the mouse with the xenograft or to a human subject and the concentration of the modified oligonucleotide in the blood or cerebral spinal fluid (CSF) of the individual is determined by calculating the area under the curve on a graph of dose over time. The optimal treatment regime of the individual can be determined by maintaining a constant blood or CSF concentration of a composition, even though the dosage required to reach that concentration would vary within an individual or between individuals.

## Claims

1. An isolated modified oligo, wherein said modified oligo comprises the nucleotide sequence of SEQ ID NO: 22, 24, 25, 26, 27, 29, 30, 31, 32, 34, 35, or 36.

2. A composition comprising the isolated modified oligo of claim 1.

3. The composition of claim 2, further comprising a cytotoxic compound.

4. The composition of claim 3, wherein said cytotoxic compound is a radioactive isotope or is a chemotherapeutic compound.

5. A composition comprising an isolated modified oligo, wherein said modified oligo comprises the nucleotide sequence of SEQ ID NO: 22, 24, 25, 26, 27, 29, 30, 31, 32, 34, 35, or 36, for use as a medicament.

6. A composition comprising an isolated modified oligo, wherein said modified oligo comprises the nucleotide sequence of SEQ ID NO: 22, 24, 25, 26, 27, 29, 30, 31, 32, 34, 35, or 36, for use in the treatment of cancer.

7. The composition for use according to claim 6, wherein said cancer is lung cancer, ovarian cancer, breast cancer, uterine cancer, head and neck cancer, pancreatic cancer, prostate cancer, renal cancer, or colon cancer.

8. The composition for use according to claim 6, wherein said composition is administered locally.

9. The composition for use according to claim 6, wherein said composition is administered systemically.

10. The composition for use according to claim 6, wherein said composition is administered topically, intravenously, intraocularly, subcutaneously, intraparitoneally, intramuscularly, intraspinally, or surgically.

11. The composition for use according to claim 6, wherein said composition is for administration to a subject who carries the LCS6 SNP mutation.

## Patentansprüche

1. Isoliertes modifiziertes Oligo, wobei das modifizierte Oligo die Nukleotidsequenz von SEQ ID NR: 22, 24, 25, 26, 27, 29, 30, 31, 32, 34, 35 oder 36 umfasst.

2. Zusammensetzung, die das isolierte modifizierte Oligo von Anspruch 1 umfasst.

3. Zusammensetzung nach Anspruch 2, die ferner eine cytotoxische Verbindung umfasst.

4. Zusammensetzung nach Anspruch 3, bei der die cytotoxische Verbindung ein radioaktives Isotop oder eine chemotherapeutische Verbindung ist.

5. Zusammensetzung, die ein isoliertes modifiziertes Oligo umfasst, wobei das modifizierte Oligo die Nukleotidsequenz von SEQ ID NR: 22, 24, 25, 26, 27, 29, 30, 31, 32, 34, 35 oder 36 umfasst, zur Verwendung als Medikament.

6. Zusammensetzung, die ein isoliertes modifiziertes Oligo umfasst, wobei das modifizierte Oligo die Nukleotidsequenz von SEQ ID NR: 22, 24, 25, 26, 27, 29, 30, 31, 32, 34, 35 oder 36 umfasst, zur Verwendung bei der Behandlung von Krebs.

7. Zusammensetzung zur Verwendung nach Anspruch 6, wobei der Krebs Lungenkrebs, Eierstockkrebs, Brustkrebs, Gebärmutterkrebs, Kopf- und Halskrebs, Bauchspeicheldrüsenkrebs, Prostatakrebs, Nierenkrebs oder Kolonkrebs ist.

8. Zusammensetzung zur Verwendung nach Anspruch 6, wobei die Zusammensetzung lokal verabreicht wird.

9. Zusammensetzung zur Verwendung nach Anspruch 6, wobei die Zusammensetzung systemisch verabreicht wird.

10. Zusammensetzung zur Verwendung nach Anspruch 6, wobei die Zusammensetzung topisch, intravenös, intraokular, subkutan, intraperitoneal, intramuskulär, intraspinal oder chirurgisch verabreicht wird.

11. Zusammensetzung zur Verwendung nach Anspruch 6, wobei die Zusammensetzung zur Verabreichung an ein Lebewesen vorgesehen ist, das die LCS6 SNP-Mutation aufweist.

## Revendications

1. Oligo modifié isolé, lequel oligo modifié comprend la séquence de nucléotides de la SEQ ID NO : 22, 24, 25, 26, 27, 29, 30, 31, 32, 34, 35, ou 36.

2. Composition comprenant l'oligo modifié isolé selon la revendication 1.

3. Composition selon la revendication 2, comprenant en outre un composé cytotoxique.

4. Composition selon la revendication 3, dans laquelle ledit composé cytotoxique est un isotope radioactif ou est un composé chimiothérapeutique.

5. Composition comprenant un oligo modifié isolé, dans laquelle ledit oligo modifié comprend la séquence de nucléotides de la SEQ ID NO : 22, 24, 25, 26, 27, 29, 30, 31, 32, 34, 35, ou 36, pour utilisation en tant que médicament.

6. Composition comprenant un oligo modifié isolé, dans laquelle ledit oligo modifié comprend la séquence de nucléotides de la SEQ ID NO : 22, 24, 25, 26, 27, 29, 30, 31, 32, 34, 35, ou 36, pour utilisation dans le traitement d'un cancer.

7. Composition pour utilisation selon la revendication 6, dans laquelle ledit cancer est un cancer du poumon, un cancer de l'ovaire, un cancer du sein, un cancer de l'utérus, un cancer de la tête et du cou, un cancer du pancréas, un cancer de la prostate, un cancer du rein, ou un cancer du côlon.

8. Composition pour utilisation selon la revendication 6, laquelle composition est administrée localement.

9. Composition pour utilisation selon la revendication 6, laquelle compositions est administrée par voie systémique.

10. Composition pour utilisation selon la revendication 6, laquelle composition administrée par voie tropique, intraveineuse, intraoculaire, sous-cutanée, intrapéritonéale, intramusculaire, intraspinale, ou chirurgicale.

11. Composition pour utilisation selon la revendication 6, laquelle composition est destinée à être administrée à un sujet qui porte la mutation LCS6 SNP.
